(19) Europäisches Patentamt
European Patent Office
Office européen des brevets

(11) **EP 1 825 859 A1**

(12) **EUROPEAN PATENT APPLICATION**

(43) Date of publication:
**29.08.2007 Bulletin 2007/35**

(51) Int Cl.:
*A61K 35/12* [(2006.01)]    *A61K 48/00* [(2006.01)]
*C12N 15/63* [(2006.01)]    *C12N 15/87* [(2006.01)]
*A61P 35/00* [(2006.01)]

(21) Application number: **06110306.5**

(22) Date of filing: **22.02.2006**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(71) Applicant: **Schuler, Gerold, Prof. Dr.**
**91080 Spardorf (AT)**

(72) Inventors:
• **Schuler, Gerold, Prof. Dr.**
**91080 Spardorf (DE)**

• **Schaft, Niels, Dr.**
**91074 Herzogenaurach (DE)**
• **Dörrie, Jan, Dr.**
**90409 Nürnberg (DE)**

(74) Representative: **von Kreisler Selting Werner Patentanwälte**
**P.O. Box 10 22 41**
**50462 Köln (DE)**

Remarks:
Claims 11-18 are deemed to be abandoned due to non-payment of the claims fees (Rule 31 (2) EPC).

(54) **Dendritic cells transiently transfected with a membrane homing polypeptide and their use**

(57) The invention provides improved methods of producing dendritic cells ("DCs") that transiently express a membrane homing peptide and optionally at least one additional antigen. These DCs have the ability to home to lymph nodes *in vivo.* In some embodiments, these DCs can be administered to a patient intravenously and can subsequently home to lymph nodes and stimulate an immune response. The methods and DCs of the invention are useful for the treatment of various diseases and disorders.

**Description**

FIELD OF THE INVENTION

The present invention relates to improved methods of transfecting dendritic cells. More particularly, the invention provides methods of producing dendritic cells that are transiently transfected with a membrane homing polypeptide. In some embodiments, the dendritic cells can deliver an antigen to a lymph node following intravenous administration.

BACKGROUND OF THE INVENTION

More than a half million Americans die of cancer annually-more than 1,500 people a day. One of every four deaths in America is from cancer. Over 2 million new cases of cancer are diagnosed annually (see, American Cancer Society, Cancer Facts and Figures 2004*). Effective treatment for patients with cancer presents a major challenge. A typical treatment regimen includes surgical resection, external beam radiation therapy, and/or systemic chemotherapy. These regimens have been partially successful in treating some kinds of malignancies, but have not produced satisfactory results in others, and they can have dramatic and undesirable side effects on a patient.

Dendritic cell-based vaccination is a relatively new approach to cancer therapy which has shown great success in some cases. Dendritic cells (DCs) are the professional antigen-presenting cells of the immune system and have the potential to dramatically stimulate an immune response. They serve as sentinel cells in virtually all tissues, including the peripheral blood, where they continuously take up and process antigens to which they are exposed. DCs subsequently migrate to draining lymph nodes, where they present antigen to lymphocytes. Immature DCs are particularly good at antigen ingestion and processing, but a productive T-cell response requires mature and fully activated DCs. Very small numbers of activated DCs are highly efficient at generating immune responses against viruses, other pathogens, and endogenous cancer.

Exploitation of the immune-regulatory capacities of dendritic cells holds great promise for the treatment of cancer. Accordingly, a significant amount of work has been done to develop DCs "vaccines" to treat various diseases and disorders. A DC vaccine comprises DCs that have been loaded with antigen, such as a tumor-associated antigen. Upon administration into patients, a DC vaccine is thought to induce an immune response against cells bearing the loaded antigen, such as an antigen-specific T-cell response against any cancer cell bearing that antigen.

However, while early results are promising, to date no DC vaccine has been approved for treatment of a disease or disorder. Careful study design and use of standardized clinical and immunological criteria are needed in order to properly assess the results of clinical trials and to provide a standardized treatment which can satisfy the rigorous requirements for regulatory approval (see, *e.g.,* Figdor et al. (2004) Nat. Med. 10: 475). Many important parameters for DC-based therapy have yet to be evaluated in clinical trials, and there is potential to improve the efficacy of DC vaccines if these parameters are optimized both individually and collectively. Thus, there is a recognized need to develop and use well-characterized and standardized DC vaccines (see, *e.g.*, Figdor et al. (2004) Nat. Med. 10: 475).

DCs can be generated in large quantities from blood-derived monocytes (referred to herein as "monocyte-derived DCs" or "moDCs") and have been used in many clinical phase I and phase II trials. In most of these trials, the DCs were administered to the patient via intra- or subcutaneous injection (see, *e.g.*, Markiewicz et al. (2004) Cancer Invest. 22: 417-434; Gilboa and Vieweg (2004) Immunol. Rev. 199: 251-263; Paczesny et al. (2003) Semin. Cancer Biol. 13: 439-447; Banchereau et al. (2001) Cell 106: 271-274; Figdor et al. (2004) Nat. Med. 10: 475-480). However, in order to reach peripheral lymphatic tissue, where antigen-specific T-cell stimulation takes place, DCs must migrate from the site of injection to the draining lymph node. Unfortunately, when DCs are administered via intra- or subcutaneous injection, their migration to regional lymph nodes (LNs) is very inefficient so that at most, about 1% to 2% of the DCs reach the regional lymph nodes (see, *e.g.,* Ridolfi et al. (2004) J. Transl. Med. 2: 27; de Vries et al. (2003) Cancer Res. 63: 12-17; Morse et al. (1999) Cancer Res. 59: 56-58). This inefficient migration is a major obstacle for effective DC vaccination (see, *e.g.*, Rosenberg et al. (2004) Nat. Med. 10: 909-915).

Another potential disadvantage of administration via intra- or subcutaneous injection involves the effect of surrounding tissues on T cells. Evidence is increasing that T cells in lymph nodes of different organs obtain a homing pattern that target the T cells to this organ after contact with the DCs, rather than allowing a more general distribution throughout the body (see, *e.g.,* von Andrian et al. (2000) New Engl. J. Med. 343: 1020-1034; Robert et al. (1999) New Engl. J. Med. 341: 1817-1828; Campbell et al. (2002) J. Exp. Med. 195: 135-141; Dudda et al. (2004) J. Immunol. 172: 857-863; Dudda et al. (2005) Eur. J. Immunol. 35: 1056-1065). Indeed, it has been reported that DC vaccination strategies in which DC were injected intracutaneously or intralymphatically resulted in effective clearance of mostly cutaneous metastases (see, *e.g.,* Nestle et al. (1998) Nat. Med. 4: 328-332; Thurner et al. (1999) J. Exp. Med. 190: 1669-1678; Banchereau et al. (2001) Cancer Res. 61: 6451-6458).

An alternative delivery system for DCs to a subject involves injection directly into the lymph node. However, this approach has significant risks and drawbacks. The volume of vaccine which can be injected into the lymph node is limited, and special equipment and experience is required to be effective. If the procedure is not performed properly, the DCs can not be delivered to the lymph node, or the structure of the lymph node can even be destroyed. It is not surprising that this method yields very variable results (see, *e.g.*, de Vries et al. (2003) Cancer Res. 63: 12-17).

Thus far, other methods of delivery have also proved ineffective. For example, intravenous injection of DCs might overcome many of these problems associated with other methods of delivery, but DCs that are intravenously injected cannot migrate directly from the blood into lymph nodes and instead accumulate first in the lung and later in the liver, spleen, and bone marrow (see, *e.g.,* Morse et al. (1999) Cancer Res. 59: 56-58). While this distribution favors a Th2-polarised immune response, this type of immune response is not beneficial in cancer treatment (see, *e.g.,* Fong et al. (2001) J. Immunol. 166: 4254-4259).

It is known that certain immune cells (such as T cells and some subpopulations of dendritic cells) are able to enter the lymph node from high endothelial venules (HEV) (see, *e.g.*, Yoneyama et al. (2005) Int. J. Hematol. 3: 204-207). This migration is made possible by various cell surface proteins, which include E-selectin, L-selectin, CCR7 and LFA-1. The CCR7 and LFA-1 proteins are also expressed on mature DCs derived from monocytes ("moDCs"), but L-selectin is not. Selectins are cell surface molecules that are important for leukocyte homing to particular tissues (see Janeway, Jr., et al., eds. (2001) Immunobiology (5th ed., Garland Publishing, New York)).

There are several types of selectins, all of which share a common core structure but which have different lectinlike domains in their extracellular portion. L-selectin is expressed on naive T cells and guides their migration from the blood into peripheral lymphoid tissues, while P-selectin and E-selectin are expressed on the vascular endothelium at sites of infection to recruit effector cells into the surrounding tissues. L-selectin binds to sulfated sialyl-Lewis[x], a carbohydrate moiety of mucinlike molecules called vascular addressins which are expressed on the surface of vascular endothelial cells. L-selectin is also known to bind to peripheral node addressin (PNAd), which is highly expressed on high endothelial venules (HEV) in lymph nodes.

L-selectin (*e.g.*, Accession No. CAB55488 or AAH56281) is also sometimes referred to as CD62L; E-selectin (*e.g.*, Accession No. AAQ67702) is also sometimes referred to as CD62E; and P-selectin (*e.g.*, Accession No. AAQ67703) is also sometimes referred to as CD62P.

Robert et al. ((2003) Gene Ther. 10: 1479-1486) have shown that murine DCs can be genetically modified by retroviral transduction so that the DCs express a chimeric E/L selectin protein on their cell surface (see also U.S. Pat. No. 6,929,792). The chimeric E/L-selectin protein combined the protease-resistant extracellular portion of E-selectin with the intracellular domain of L-selectin in order to avoid shedding from the protease-rich DC cell surface while retaining the binding specificity of L-selectin. When DCs expressing this chimeric selectin are injected intravenously into a subject, they can bind to peripheral node addressin and migrate through the HEV to lymph nodes.

However, this method has several significant disadvantages which prevent its use for treatment of humans. First, cells modified by retroviral transduction are not suitable for therapy in humans, particularly from a regulatory standpoint (see, *e.g.*, Haviernik and Bunting (2004) Curr. Gene Ther. 4: 263-276). The largest drawback of using retroviral transduction is that the provirus can integrate randomly into the genome of the transduced cells, possibly disrupting the expression of important genes, such as, for example, those involved in cell cycle control. A well-known example of this unfortunate event occurred in a gene therapy clinical trial for severe combined immunodeficiency (SCID). In this trial, autologous hematopoietic stem cells were transduced with a retroviral vector containing a gene encoding the common γ chain, which is defective in SCID patients; in at least two instances, the provirus integrated in the LMO-2 oncogene, causing leukemia-like symptoms in the patients (see, *e.g.,* Buckley (2002) Lancet 360: 1185-1186; Hacein-Bey-Abina et al. (2002) New Engl. J. Med. 346: 1185-1193; Marshall (2002) Science 298: 34-35).

Another drawback of the use of retroviral transduction to produce DCs is that the efficiency of transfection is variable and retroviral transduction can only be used in dividing cells, such DCs derived from proliferating CD34[+] cells, and not in DCs derived from non-proliferating monocytes (see, *e.g.*, Ardeshna et al. (2000) Br. J. Haematol. 108: 817-824). However, isolation of CD34[+] stem cells is a time-consuming and complicated procedure and is also a burden for the patient, so other alternatives are often preferred.

Alternatives that might be used to transduce non-dividing dendritic cells include lentiviral transduction or adenoviral transduction. However, lentiviral transduction results in heterogeneous expression of the introduced molecule (see, *e.g.,* Dullaers et al. (2004) Mol. Ther. 10: 768-779; Breckpot et al. (2003) J. Gene Med. 5: 654-667; Sumimoto et al. (2002) J. Immunol. Meth. 271: 153-165). Adenoviral transduction (see, *e.g.,* Korokhov et al. (2005) Cancer Biol. Ther. 4: 289-294; Ophorst et al. (2004) Vaccine 22: 3035-3044; Rea et al. (2001) J. Immunol. 166: 5236-5244) also results in heterogeneous expression of the introduced molecule (see, *e.g.,* Cho et al. (2003) Vaccine 22: 224-236), and may even lead to induction of regulatory T cells instead of an efficient anti-cancer response (see, *e.g.,* Lundqvist et al. (2005) J. Immunother. 28: 229-235).

Another method which has been used to transduce cells is RNA electroporation. This method ensures that only transient protein expression will occur, since chromosomal integration is impossible; thus, the method only temporarily changes the properties of the cell. RNA electroporation is well-suited to applications where only transient expression of certain molecules is necessary, such as targeting of DCs from blood to lymph nodes and presentation of antigen by DCs. RNA electroporation has been used with DCs, although results have generally been mixed (see, *e.g.,* Van Tendeloo et al. (1998) Gene Ther. 5: 700-707). However, if an optimized electroporation method is employed, high transfection rates of around 95% can be obtained (see, *e.g.,* Schaft et al. (2005) J. Immunol. 174: 3087-3097).

So far, RNA electroporation has only been used in limited circumstances, such as to deliver antigen (Ag) to DCs or to increase the DC's stimulatory capacity (see, *e.g.*, Abdel-Wahab et al. (2005) J. Surg. Res. 124: 264-273; Dannull et al. (2005) Blood 105: 3206-3213; Grunebach et al. (2005) Cancer Gene Ther. 12: 749-756; Cisco et al. (2004) J. Immunol. 172: 7162-7168). While the proteins expressed in these studies were shown to be functional as signaling receptors or proteins involved in certain signaling cascades, fairly low expression levels were reported. However, in order to obtain DCs that are capable of migrating from the blood to peripheral lymph nodes, it was believed that much higher expression levels of protein on the cell surface would be necessary, and that such high expression levels would be difficult if not impossible to achieve using RNA transfection.

Thus, there remains a need for a method of producing DCs that can home to lymph nodes following intravenous administration and that can be used as vaccines for the treatment of various diseases and disorders.

SUMMARY OF THE INVENTION

The invention provides improved methods of producing dendritic cells ("DCs") that transiently express a membrane homing peptide and optionally at least one additional antigen. These DCs have the ability to home to lymph nodes *in vivo.* In some embodiments, these DCs can be administered to a patient intravenously and can subsequently stimulate an immune response to an antigen of interest. The methods and DCs of the invention are useful for the treatment of various diseases and disorders.

BRIEF DESCRIPTION OF THE DRAWINGS

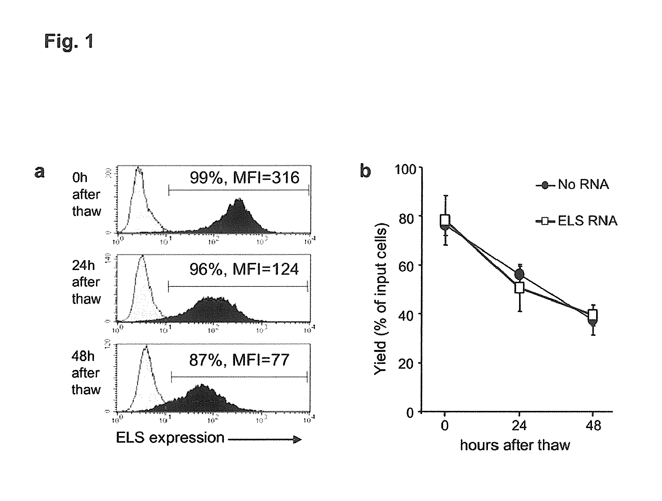

Figure 1 demonstrates that expression of E/L-selectin in DC cells is high following electroporation of E/L-selectin RNA. Mature DCs were electroporated with or without E/L-selectin (ELS) RNA and cryopreserved after 4 h; cells were then thawed and assayed for ELS expression by FACS analysis at 0h, 24h, and 48h after thawing. Panel (a) shows ELS expression of DCs electroporated with ELS RNA (black histogram) and control DCs that were mock-electroporated (grey histogram). For each timepoint, panel (a) also provides the percentage of ELS-positive cells and mean fluorescence intensity (MFI). The data shown was representative of three independent standardized experiments. Panel (b) shows the DC yield from DCs electroporated with ELS RNA (white squares) and mock-electroporated DCs (black circles). "Yield" is the percentage of living cells after electroporation and cryopreservation in comparison to the number of cells prior to electroporation; viability of cells was determined by trypan-blue staining. The data shown represents an average of three independent standardized experiments +/- standard error of the mean (SEM).

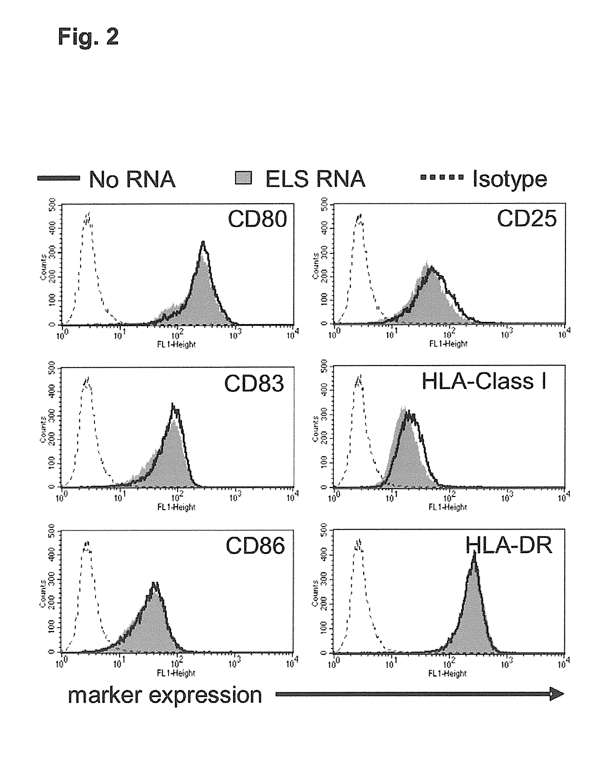

Figure 2 demonstrates that DCs transfected with ELS RNA retained their mature phenotype. Mature DCs were electroporated with E/L-selectin (ELS) RNA (grey histogram) or without ELS RNA (black line), allowed to rest for 4 hours, cryopreserved, thawed, and stained for the characteristic maturation-surface markers CD25, CD80, CD83, CD86, HLA class I, and HLA-DR. Dashed lines represent isotype controls for each marker, respectively. The data shown are representative of three independent standardized experiments.

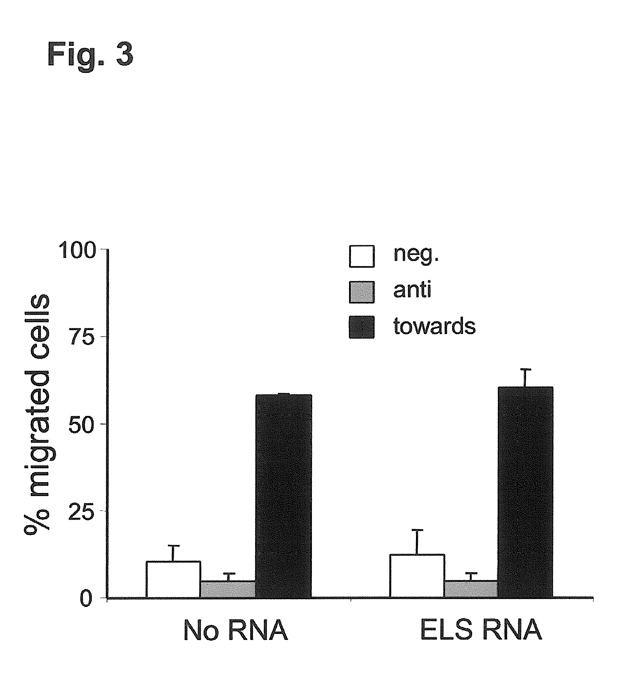

Figure 3 demonstrates that DCs transfected with E/L-selectin retain their CCR7-mediated migratory capacity. DCs were electroporated with E/L selectin RNA ("ELS RNA") or mock-electroporated without RNA ("No RNA") and then tested for their CCR7-mediated migratory capacity towards medium containing CCL19 in a standard transwell migration assay (black bars; "towards") (see Example 2). Spontaneous migration was measured by incubating cells in a transwell without CCL19 in the upper or lower compartment (white bars; "neg."), and another control included CCL19 in the upper compartment (grey bars; "anti"). An average (+/- SEM) of three standardized experiments is shown.

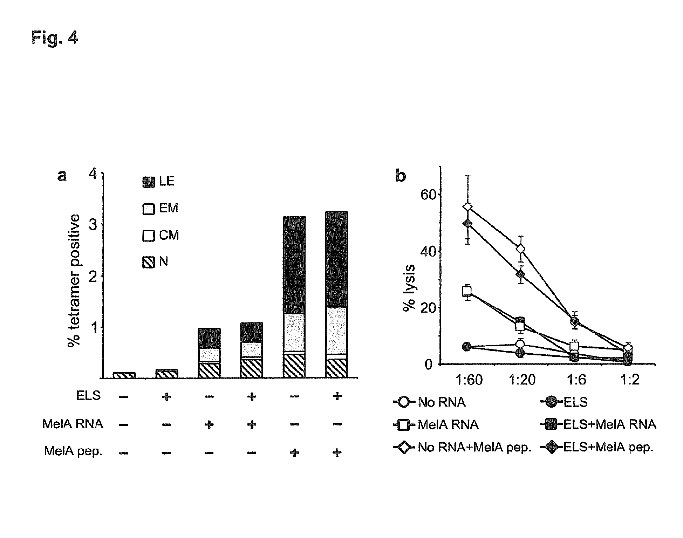

Figure 4 demonstrates that DCs transfected with E/L-selectin retain their capacity to stimulate naive CD8[+] T-cells. DCs were electroporated without RNA (open circles; "No RNA"), with E/L-selectin RNA alone (black circles; "ELS"), with MelanA RNA alone (open squares; "MelA RNA"), or with MelanA in combination with E/L-selectin RNA (black squares; "ELS+Me1A RNA"), and were used to stimulate autologous naive CD8[+] cells. In addition, some DCs that had been mock-electroporated or electroporated with E/L-selectin RNA alone were loaded with MelanA-derived analogue peptide (MelA pep.) and used for stimulation of naive CD8[+] T cells. After one week of stimulation, MelanA/A2-tetramer-binding CD8[+] T cells were evaluated for phenotype (Panel (a)), and cytolytic capacity (Panel (b)). Functional T cell phenotypes were assigned as follows: lytic effectors ("LE"; CD45RA[+]/CCR7[--]); effector memory ("EM"; CD45RA$^-$/CCR7$^-$); central memory ("CM"; CD45RA$^-$/CCR7$^+$); naive ("N"; CD45RA$^+$/CCR7$^+$). Cytolytic capacity (b) was determined in a standard Cr$^{51}$-release assay using targets of T2 cells loaded with the MelanA analogue peptide or the irrelevant gp100 peptide. Data shown in Panel (b) is percent lysis of T2 target cells loaded with MelanA analogue peptide; for all T cell populations tested, lysis of T2 cells loaded with gp100 peptide was less than 10%. Target:effector ratio (T:E) is shown on the horizontal axis. Data shown are from one representative experiment of three independent standardized experiments.

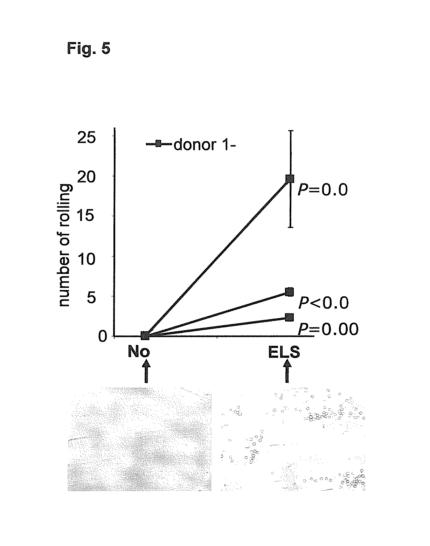

Figure 5 demonstrates that DCs transfected with E/L-selectin RNA gain the ability to roll on sialyl-Lewis$^x$-coated slides. DCs from three human donors were electroporated with E/L selectin RNA ("ELS RNA") or were electroporated without RNA ("No RNA") and cryopreserved. They were then thawed and evaluated for their ability to roll in a parallel plate flow chamber using slides coated with sialyl-Lewis$^x$ (see Example 4). Perfusion was performed at 20°C using a pulse-free pump at a shear rate of 1.04 dyne/s$^2$. During perfusion, microscopic phase-contrast images were recorded in real time (one representative image is depicted (10x objective)). After 10 minutes of perfusion, four different microscopic fields were recorded and the numbers of rolling (i.e., adhering) cells were counted for each field. Values shown are an average (+/-SEM) of cell numbers of the four fields for each donor. P-values are provided, indicating the statistical significance of the data (Student's T-test).

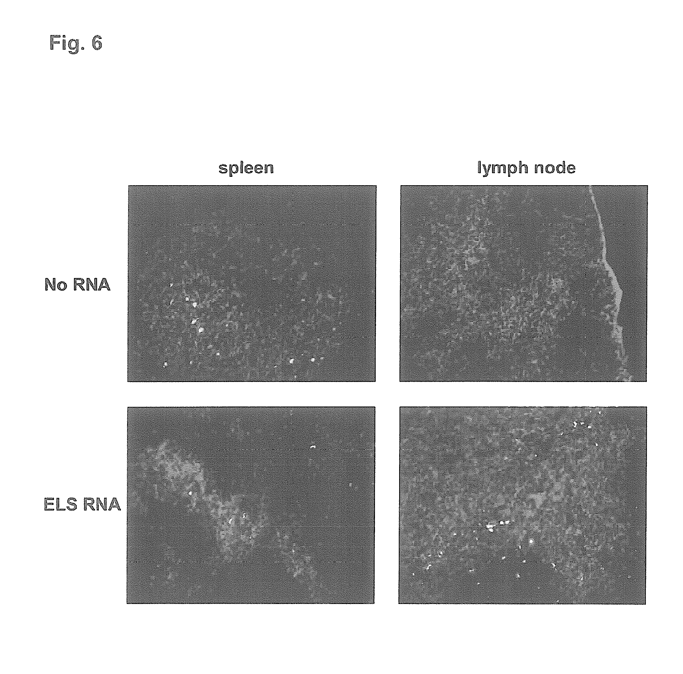

Figure 6 demonstrates that DCs which have been transfected with E/L selectin are able to transmigrate into peripheral lymph nodes *in vivo.* Murine dendritic cells (C57/B6, female) were electroporated with E/L-selectin RNA ("ELS RNA") or without RNA ("No RNA"), stained with 5-chloromethylfluorescein diacetate (CMFDA), and injected into the tail vein of C57/B6 female mice. Sixteen hours after injection, the mice were sacrificed and cryosections were made of spleen and peripheral lymph nodes. T-cell areas were stained with CD90.2 (Thy1.2)/alexa555 and cryosections were analyzed by fluorescence microscopy. Photographs shown are of representative areas of the organs from one experiment out of four experiments (100 x objective).

## DETAILED DESCRIPTION OF THE INVENTION

The invention provides improved methods of producing dendritic cells ("DCs") which transiently express a membrane homing peptide and have the ability to home to lymph nodes *in vivo.* In some embodiments, the invention provides improved methods of RNA electroporation which can produce a population of DCs that are capable of homing to lymph nodes, for example, following intravenous injection into a patient. DCs produced by the methods of the invention are also provided; these DCs do not contain retroviruses, and the expression of the membrane homing peptide by the DCs does not interfere with the expression or function of other cell surface proteins. Thus, DCs of the invention can be used as vaccines for the treatment of various diseases and disorders and can be administered by intravenous injection. Accordingly, the invention also provides methods of treating a patient with DCs.

In some embodiments, DCs are transfected so as to transiently express a membrane homing polypeptide and at least one other antigen (sometimes referred to herein as an "antigen of interest"). These dendritic cells can home to the lymph node following intravenous administration, thereby delivering said at least one other antigen to the lymph node. There, the DCs can present the antigen to T cells and stimulate an immune response.

The improved transfection methods of the invention result in high transfection efficiency and yield of DCs, even following cryopreservation, and also permit very high levels of expression from transfected nucleic acid(s). In this manner, the present invention provides for the first time DCs that functionally express membrane homing peptides, as demonstrated by the ability of DCs to "tether" (*i.e.*, "stick") and/or "roll" on surfaces coated with a ligand of the membrane homing peptide (see, *e.g.*, Example 4). Accordingly, in some embodiments, the invention provides an isolated dendritic cell which has been transiently transfected with RNA encoding a membrane homing polypeptide wherein the dendritic cell can roll on a surface coated with a ligand of that membrane homing polypeptide. Thus, in some embodiments, an isolated DC is also provided.

The ability of DCs to "tether," "stick," and/or "roll" to certain surfaces *in vitro* is correlated with the ability of DCs *in vivo* to move from the blood into a lymph node following intravenous administration. For DCs expressing selectin, these tethering, sticking, and rolling behaviors can be evaluated in a parallel plate flow chamber assay using surfaces coated with sialyl-Lewis$^x$, as demonstrated in working Example 4 (see also Robert et al. (2003) Gene Therapy 10: 1479-1486). Thus, the methods of the invention produce DCs that show increased tethering, sticking, and/or rolling on surfaces coated with an appropriate ligand in comparison to appropriate control cells, whether *in vitro* or *in vivo.*

Specifically, by "tethering" or "sticking" and "rolling" is intended that DCs that have been transfected with RNA encoding a membrane homing peptide, and that are in suspension in a medium that flows over an adequate test surface will frequently bind to the surface and roll along with a velocity that is no greater than 20% of the velocity of the flow of the medium. An adequate test surface is a surface coated with a substance or ligand to which the introduced membrane homing peptide is capable of binding, and may be *in vitro* or *in vivo.* Thus, an individual DC is considered to exhibit "tethering" or "sticking" and "rolling" behavior if it binds to and rolls on a surface with a velocity that is no greater than 20% of the velocity of the flow of the medium. A population of cells is considered to exhibit "tethering" or "sticking" and "rolling" behavior if 10 times more cells are observed to bind to and roll on a defined area of the test surface than the number of control cells observed to roll under similar conditions. Thus, for example, a population of DCs exhibits tethering and rolling behavior if 10 times more DCs bind to and travel along a surface coated with sialyl-Lewis[x] under a shear force of between 1 to 5 dyn/cm$^2$ at a speed less than 20% of the flow in comparison to appropriate control cells under the same circumstances.

For this and other assays described herein, an appropriate control cell is one which has not been transfected with RNA encoding a membrane homing peptide. Thus, for example, an appropriate control cell may be a DC that has been mock-electroporated, or it may be a DC that has been electroporated with RNA that does not encode a membrane homing peptide. One of skill in the art is familiar with the selection and preparation of appropriate controls for any particular assay.

The improved methods of the invention provide many DCs that will exhibit the desired behaviors (i.e., tethering, sticking, and/or rolling behavior when the DCs have been transfected with a membrane homing peptide and are assayed in a parallel plate flow chamber assay using surfaces coated with a ligand of the membrane homing peptide). Thus, for example, in a population of DCs that have been transfected according to the methods of the invention, (e.g., electroporated with RNA coding for a selectin), at least 10 times more of the cells will tether, stick, and roll on an appropriate test surface (e.g., on a slide coated with sialyl-Lewis[x] or a high endothelial venule) than in a control population (e.g., mock-transfected DC or DC transfected with an irrelevant RNA as one coding for EGFP).

DCs of the invention are capable of "homing" to a lymph node, i.e., moving from the blood into at least one lymph node following intravenous injection. Thus, when DCs of the invention are injected intravenously, it is possible to detect DCs of the invention in at least one lymph node at a later time point, such as at least 1 hour, 2 hours, 4 hours, 8 hours, 18 hours, 24 hours, 48 hours, or more following the time of injection. DCs of the invention may migrate out of the blood in high endothelial venules ("HEVs").

The methods of the invention for the first time provide a DC population which exhibits altered migratory behavior as a result of the expression of a surface receptor where that expression is the result of transient transfection rather than of permanent genetic modification of the cell. In other words, DC populations produced by the methods of the invention acquire new desirable attributes while avoiding a need for heritable genetic modification of the genome. In this manner, the invention makes possible new and improved DC vaccines for a variety of uses.

Because the methods of the invention provide DCs that are both quantitatively and qualitatively superior to those produced by previously known methods, it may be possible to treat diseases and disorders that were previously refractory to treatment with DCs, such as, for example, metastatic melanoma. It will be appreciated that while the invention can be used to treat existing cancers, it can also be used to prevent cancer. Another advantage provided by the invention is that the DCs of the invention are not targeted only to a single lymph node but instead will home to multiple lymph nodes, which is expected to increased contact between DCs and T cells and thus to produce a more efficient stimulation of antigen-specific T cells.

Yet another advantage provided by the invention is that DCs of the invention can be administered to a patient by intravenous injection. It has been reported that administration of DCs by intra- or subcutaneous injection allows the DCs only to enter skin-draining lymph nodes and so may impart a homing pattern that directs the resulting immune response to skin and skin-associated diseases and disorders, rather than allowing a more general immune response. Intravenous administration, in contrast, allows the DCs to enter lymph nodes draining a broad spectrum of tissues and organs and thus could be more efficient for the induction of an immune response in and around organs in addition to the skin.

Moreover, the methods of the invention provide DCs which retain three features of DCs that are important for DC vaccines: a mature DC phenotype (i.e., high expression of CD80, CD83, CD86, CD25, HLA-class I, and HLA-DR expression, as demonstrated in Example 1); maintenance of normal CCR7-mediated migration capacity (as demonstrated in Example 2); and maintenance of antigen-specific T cell stimulation capacity (as demonstrated in Example 3).

The term "dendritic cells" (DCs) refers to a diverse population of morphologically similar cell types found in a variety of lymphoid and non-lymphoid tissues (see, e.g., Steinman (1991) Ann. Rev. Immunol. 9: 271-296). Dendritic cells are the most potent and preferred antigen presenting cells (APCs). Dendritic cells can be differentiated from monocytes or other precursor cells and possess phenotypes distinct from these precursors. For example, monocytes express CD14 antigen, while mature dendritic cells do not. Also, mature dendritic cells are not phagocytic, whereas monocytes are strongly phagocytosing cells.

Some of the cell surface markers characteristic of the different stages of dendritic cell maturation of the dendritic cells are summarized in Table I below. However, surface markers can vary depending upon the maturation process. Thus, as used herein, "dendritic cell" refers to a cell that expresses at least one of the markers that is characteristic of a mature dendritic cell and does not express at least one of the markers that is characteristic of an immature dendritic cell or a monocyte. For example, a mature dendritic cell could express CD83 but not CD14 at high levels.

Table I

| Cell type | Surface markers |
| --- | --- |
| Hematopoietic stem cell | CD34+ |
| Monocyte | CD14++, DR+, CD86+, CD16+/-, CD54+, CD40+ |
| Immature dendritic cell | CD14+/-, CD16-, CD80+/-, CD83-, CD86+, CD1a+, CD54+, DQ+, DR++ |
| Mature dendritic cell | CD14-, CD83++, CD86++, CD80++, DR+++, DQ++, CD40++, CD54++, CD1a +/- |

Mature DCs are currently preferred to immature DCs for immunotherapy. Only fully mature DCs lack GM-CSF Receptor (GM-CSF-R) and remain stably mature upon removal of (*i.e.,* in the absence of) GM-CSF. Mature DCs have been shown to be superior to immature DCs in inducing T cell responses *in vitro* and *in* vivo, can provide all the signals necessary for T cell activation and proliferation. In contrast, immature DCs are reported to induce regulatory T cells and thereby to induce tolerance *in vitro* as well as *in vivo* (see, *e.g.*, Jonuleit et al. (2000) Exp. Med. 192:1213; Dhodapkar et al. (2001) Exp. Med. 193:233). Mature dendritic cells take up and present antigen to T-lymphocytes *in vitro* or *in vivo.* The antigen-presenting DCs of the invention and/or T cells educated by these DCs *(i.e.,* T cells to which these DCs have presented antigen and which will recognize that antigen as a result) have many applications, including research, diagnostics, and therapy.

Mature DCs are difficult to extract from tissues, and it is difficult to isolate mature dendritic cells from peripheral blood because less than 1% of the white blood cells belongs to this category. Accordingly, there has been much research directed to methods of generating mature dendritic cells from other sources. Methods have been developed by which immature DCs can be isolated or prepared from a suitable tissue source containing DC precursor cells and differentiated *in vitro* to produce mature DCs. For example, suitable tissue sources include: bone marrow cells, peripheral blood progenitor cells (PBPCs), peripheral blood stem cells (PBSCs), and cord blood cells. Preferably, the tissue source is a peripheral blood mononuclear cell (PBMC). The tissue source can be fresh or frozen. In some methods, the cells or tissue source are pre-treated with an effective amount of a growth factor that promotes growth and differentiation of non-stem or progenitor cells, which are then more easily separated from the cells of interest. These methods are known in the art and described briefly, for example, in Romani et al. (1994) J. Exp. Med. 180: 83 and Caux et al. (1996) J. Exp. Med. 184: 695-706.

In some embodiments of the invention, immature DCs are isolated from peripheral blood mononuclear cells (PBMCs) or a subpopulation thereof. PBMCs can be prepared from leukapheresis products or whole blood of healthy donors by density centrifugation. The PBMCs are treated with an effective amount of granulocyte macrophage colony stimulating factor (GM-CSF) in the presence or absence of interleukin 4 (IL-4) and/or IL-13, so that the PBMCs differentiate into immature DCs. In some embodiments, PBMCs are cultured in the presence of GM-CSF and IL-4 for about 4-7 days, preferably about 5-6 days, to produce immature DCs. The first maturation signal can be given at day 4, 5, 6, or 7, and most preferably at day 5 or 6. In addition, GM-CSF as well as IL-4 and/or IL-13 may be present in the medium at the time of the first and/or second signaling. Such methods are known in the art.

DCs can be generated from frequent but non-proliferating CD14[+] precursors (monocytes) in peripheral blood by culture in medium containing GM-CSF and IL-4 or GM-CSF and IL-13 (see, *e.g.,* WO 97/29182; Sallusto and Lanzavecchia (1994) J. Exp. Med. 179:1109; Romani et al. (1994) J. Exp. Med. 180: 83; Berger et al. (2002) J. Immunol. Meth. 268: 131-140. Others have reported that DCs generated by this approach appear rather homogenous and can be produced in an immature state or a mature (i.e., fully differentiated) state. Fully mature and irreversibly stable DCs can be obtained using autologous monocyte conditioned medium to stimulate maturation (see, *e.g.*, Romani et al. (1996) J. Immunol. Meth. 196: 137-151; Bender et al. (1996) J. Immunol. Meth. 196: 121) or can be obtained using a defined maturation mixture (see, *e.g.,* Jonuleit et al. (1997) Eur. J. Immunol. 27: 3135; Schaft et al. (2005) J. Immunol. 174: 3087-3097).

While DCs of the present invention are typically prepared from PBMCs or monocytes, dendritic cells may also be prepared from other cells such as, for example, CD34[+] stem cells. Many methods are known in the art for the isolation and expansion of CD34[+] stem cells and for their differentiation into dendritic cells (see, *e.g.*, U.S. Pat. No. 5,199,942).

CD34[+] stem cells can be isolated from bone marrow cells or by panning the bone marrow cells or other sources with antibodies which bind unwanted cells, such as, for example, CD4[+] and CD8[+] cells (T cells) and CD45[+] cells (panB cells) (see, *e.g.,* Inaba et al. (1992) J. Exp. Med. 176: 1693-1702). Human CD34[+] cells can be obtained from a variety of sources, including cord blood, bone marrow explants, and mobilized peripheral blood. Purification of CD34[+] cells can be accomplished by antibody affinity procedures (see, e.g., Paczesny et al. (2004) J. Exp. Med. 199: 1503-11; Ho et al. (1995) Stem Cells 13 (suppl. 3): 100-105; Brenner (1993) J. Hematother. 2: 7-17; and Yu et al. (1995) Proc. Nat'l. Acad.

Sci. 92: 699-703).

CD34+ stem cells can be differentiated into dendritic cells by incubating the cells with the appropriate cytokines. Such methods are known in the art. For example, Inaba et al. ((1992) J. Exp. Med. 176: 1693-1702) described the *in vitro* differentiation of murine stem cells into dendritic cells by incubating the stem cells with murine GM-CSF. In brief, isolated stem cells are incubated with between 1 and 200 ng/ml murine GM-CSF, preferably about 20 ng/ml GM-CSF in standard RPMI growth medium which is replaced with fresh medium about once every other day. IL-4 is optionally added in similar ranges. After approximately 5-7 days in culture, many of the cells are dendritic, as assessed by expression of surface markers and cell morphology. The dendritic cells can then be isolated by florescence activated cell sorting (FACS) or by other methods known in the art.

Human CD34+ hematopoietic stem cells can also be differentiated *in vitro* by culturing the cells with human GM-CSF and TNF-$\alpha$ (see, *e.g.*, Szabolcs, et al. (1995) J. Immunol. 154: 5851-5861). Human GM-CSF is used in similar ranges, and TNF-$\alpha$ can also added in about the same ranges to facilitate differentiation. Optionally, SCF or another proliferation ligand (*e.g.*, Flt3) is added in similar dose ranges to differentiate DCs. WO 95/28479 also discloses a process for preparing dendritic cells by isolating peripheral blood cells and enriching for CD34+ blood precursor cells followed by expansion with a combination of hematopoietic growth factors and cytokines.

European Patent Publication EP-A-0 922 758 discloses the production of mature dendritic cells from immature dendritic cells derived from pluripotential cells having the potential of expressing either macrophage or dendritic cell characteristics. The method requires contacting the immature dendritic cells with a dendritic cell maturation factor containing IFN-$\gamma$. European Patent Publication EP-B-0 633930 teaches the production of human dendritic cells by first culturing human CD34+ hematopoietic cells (i) with GM-CSF, (ii) with TNF-$\alpha$ and IL-3, or (iii) with GM-CSF and TNF-$\alpha$ to induce the formation of CD1a+ hematopoietic cells. U.S. Patent Publication No. 2004/0152191 discloses the maturation of dendritic cells by contacting them with RU 41740. U.S. Patent Publication No. 2004/0146492 teaches a process for producing recombinant dendritic cells by transforming hematopoietic stem cells followed by differentiation of the stem cells into dendritic cells by culture in medium containing GM-CSF. U.S. Patent Publication No. 2004/0038398 discloses methods for the preparation of substantially purified populations of DCs and monocytes from the peripheral blood of mammals. Myeloid cells are isolated from a mammal and DCs are separated from this population to yield an isolated subpopulation of monocytes. DCs are then enriched by negative selection with anti-CD2 antibodies to remove T cells. PCT/US05/036304, the contents of which are herein incorporated by reference, discloses methods for maturing dendritic cells using CD40L, interferon, PGE2 and optionally TNF-$\alpha$.

As is known in the art, dose ranges for differentiating stem cells and monocytes into dendritic cells are approximate. Cytokines from different suppliers and even from different lots from the same supplier vary in their activity. One of skill can easily titrate each cytokine which is used to determine the optimal dose for any particular cytokine.

DCs of the invention may be derived from cells obtained from a human donor, or may be obtained from cells obtained from any animal donor, such as, for example, from a mouse or a dog. To increase the number of dendritic precursor cells in animals, including humans, subjects can be pre-treated with substances which stimulate hematopoiesis (*i.e.*, hematopoietic factors). Such substances include but are not limited to G-CSF and GM-CSF. An effective amount of hematopoietic factor to be administered may be determined by monitoring the cell differential of individuals to whom the factor is being administered. Typically, dosages of factors such as G-CSF and GM-CSF will be similar to the dosage used to treat individuals recovering from treatment with cytotoxic agents. As an example, GM-CSF or G-CSF can be administered for 4 to 7 days at standard doses prior to removal of source tissue to increase the proportion of dendritic cell precursors. U.S. Patent No. 6,475,483 teaches that dosages of G-CSF of 300 micrograms daily for 5 to 13 days and dosages of GM-CSF of 400 micrograms daily for 4 to 19 days result in significant yields of dendritic cells.

Unless otherwise indicated, the practice of the present invention employs conventional techniques of molecular biology, including recombinant techniques, microbiology, cell biology, biochemistry and immunology, all of which are known in the art. For example, various techniques useful in the practice of the invention are described in the following publications: Sambrook et al. (1989) MOLECULAR CLONING: A LABORATORY MANUAL, 2nd edition (1989); CURRENT PROTOCOLS IN MOLECULAR BIOLOGY (Ausubel et al. eds. (1987)); the series METHODS IN ENZYMOLOGY (Academic Press, Inc.); PCR: A PRACTICAL APPROACH (M. MacPherson et al. IRL Press at Oxford University Press (1991)); PCR 2: A PRACTICAL APPROACH (MacPherson, Hames and Taylor eds. (1995)); ANTIBODIES: A LABORATORY MANUAL (Harlow and Lane eds. (1988)); USING ANTIBODIES: A LABORATORY MANUAL (Harlow and Lane eds. (1999)); and ANIMAL CELL CULTURE (Freshney ed. (1987)).

As used herein, "to transfect" or "transfection" refers to the introduction of one or more exogenous nucleic acids or polynucleotides into the cell. Transfection includes introduction in such a manner that a protein encoded by the nucleic acid or polynucleotide can be expressed. Transfection methods are known in the art and include a variety of techniques such as electroporation, methods using protein-based, lipid-based and cationic ion based nucleic acid delivery complexes, viral vectors, "gene gun" delivery, and various other techniques.

In preferred embodiments of the invention, a polynucleotide introduced into a DC does not genetically modify the DC and is not stably maintained. The term "genetically modified" means containing and/or expressing a foreign gene or nucleic acid sequence which in turn modifies the genotype of the cell or its progeny, possibly also altering the phenotype of the cell. In other words, it refers to any addition, deletion or disruption to a cell's endogenous nucleotides. Stable maintenance of an introduced polynucleotide typically requires that the polynucleotide either contains an origin of replication compatible with the host cell or integrates into a replicon of the host cell such as an extrachromosomal replicon (*e.g.*, a plasmid) or a nuclear or mitochondrial chromosome.

As used herein, "transiently transfected" refers to a cell that has been transformed in such a way that progeny of the transformed cell do not inherit the transformed genetic material (*e.g.*, nucleic acid or polynucleotide). The genetic material may be transcribed into RNA, and a protein encoded by the genetic material may be expressed. Such expression is referred to herein as transient expression. Normally, transient expression is accomplished by not incorporating the transfected genetic material into the chromosome.

In some embodiments of the invention, dendritic cells are transiently transfected using RNA electroporation. Generally, mRNA does not become a permanent part of the genome of the cell, either chromosomal or extrachromosomal. Any other methods that could be used to transiently express a desired protein are also contemplated within the scope of the invention. The methods do not involve permanent alteration of the genome (*i.e.,* do not result in heritable genetic change to the cell) and thus avoid the disadvantages associated with transfection using viruses, such as, for example, retroviruses and adenoviruses.

While RNA electroporation is known in the art, the levels of expression that could be obtained with previously known methods were not high enough to produce DC populations with functional levels of surface receptor expression. The methods of the present invention include RNA electroporation methods which have been optimized so that expression levels in DCs produced by the method are similar to the levels obtained with viral transfection, yet the disadvantages of viral transfection are avoided. That is, the methods of the invention provide high transfection efficiency such that at least 60%, 70%, 80%, 90%, or 95% or more of the cells treated according to the method contain at least some of the nucleic acid or polynucleotide used for transfection. The methods of the invention also provide a high yield of at least 60%, 70%, 80%, 90%, 95%, or more viable and transfected dendritic cells, either before or after cryopreservation (see, *e.g.*, working Example 1). Thus, also provided by this invention are enriched populations of mature DCs prepared by any of the methods described herein. In some embodiments, the dendritic cells are mature prior to transfection and/or cryopreservation. In another aspect, the invention provides a method for storing an enriched population of mature DCs, comprising contacting an enriched dendritic cell population of the invention with a suitable cryopreservative under suitable conditions.

In an effort to develop improved electroporation methods, a number of parameters were altered and evaluated. For these experiments, the RNA used for electroporation encoded a test protein (enhanced GFP, or "EGFP"). Generally, under the conditions evaluated, protein expression was proportional to the concentration of RNA used but was independent of the concentration of cells up to a concentration of $60 \times 10^6$ DC/ml. For both human and murine DCs, RNA was generally used at a final concentration of or about 150 micrograms per milliliter, such as, for example, at a concentration of or about 100, 125, 150, 175, or 200 micrograms per milliliter. For human DCs, the RNA was incubated with the cells in the cuvette prior to electroporation for about 1, 2, or 3 minutes. For murine DCs, the RNA was generally not preincubated with the cells in the cuvette prior to electroporation, although a preincubation step could optionally be included.

Typically, cells were resuspended in OptiMEM without phenol-red (Gibco-BRL, Long Island, USA) at a concentration of about $4\text{-}6 \times 10^7$ cells/ml (human) or $4\text{-}10 \times 10^7$ cells/ml (mouse), although other cell concentrations can be used. Protein expression was proportional to the pulse time between 0.5 ms and 2 ms, but at 2 ms and higher, a greater percentage of the human cells died. Murine DCs tolerated a pulse time of 2 ms well but died at 5 ms. Higher voltages were generally better because the mean fluorescence intensity (MFI)--which is proportional to the amount of expressed EGFP increased with increasing voltage, but higher voltages also increased cell death, and the voltage used was less important to success compared to the other parameters.

The improved RNA electroporation methods of the invention include optimization of the pulse time to a value that results in high levels of expression while not killing too many DCs. Particularly, improved electroporation conditions for human DCs included the use of a 4 mm cuvette with a charge of between 400V and 600V and a square wave pulse of between 0.8 ms and 2 ms at room temperature in Optimem medium.

Field strength is measured by dividing the charge by the gap width of the cuvette; thus, for example, the use of a 500V charge with a 4 mm cuvette results in a field strength of 125 V/mm, a field strength that could also be obtained using different combinations of charge and cuvette widths. Accordingly, other combinations of charge and cuvette width that result in a field strength of between 100 V/mm and 150 V/mm are also provided by the invention, such as, for example, combinations that result in a field strength of 100, 120, 125, 130, 140, or 150 V/mm. Thus, for example, with a 4 mm cuvette, the improved electroporation conditions include a charge of or about 400V, 450V, 500V, 550V, or 600V, and a square wave pulse of or about 0.8, 1, 1.2, 1.4, 1.6, 1.8, or 2 ms in length.

The optimal electroporation conditions for murine DCs included the use of a 500V charge with a 4 mm cuvette (*i.e.*, a field strength of 125 V/mm) and a 2 millisecond square wave pulse at room temperature in Optimem medium. Accordingly, for electroporation of murine DCs, other combinations of charge and cuvette width that result in a field strength of between 100 V/mm and 150 V/mm are also provided by the invention, such as, for example, combinations that result in a field strength of 100, 120, 125, 130, 140, or 150 V/mm. Thus, for example, with a 4 mm cuvette, the improved electroporation conditions include a charge of or about 400V, 450V, 500V, 550V, or 600V, and a square wave pulse of or about 0.8, 1, 1.2, 1.4, 1.6, 1.8, 2, 2.2, 2.4, 2.6, 2.8, 3, 3.2, 3.4, 3.6, 3.8, 4, 4.2, 4.4, 4.6, 4.8, or 5 ms in length.

Square wave pulses used for electroporation are distinct from exponential decay pulses and provide superior results in the methods of the invention. For exponential decay pulses, generally, a capacitor is discharged into the sample and the voltage across the electrodes rises rapidly to the peak voltage and then declines over time with an exponential decay waveform (see, *e.g.*, Bio-Rad Instruction Manual for the Gene Pulser Xcell™ Electroporation System, Bio-Rad, Munich Germany). Exponential decay pulses can be described by the following formula:

$$V_t = V_o[e^{-(t/RC)}]$$

where $V_t$ is the voltage at time = t milliseconds after the pulse, $V_o$ is the initial voltage in the capacitor, e is the base of the natural logarithm, R is the resistance of the circuit (expressed in ohms), and C is the capacitance (expressed in microfarads).

For square wave pulses, the pulse length *(i.e.,* the length of time for which cells are exposed to the electric field) is controlled directly by setting the time for which the cells are exposed to the electric field. Square wave pulses can be generated by truncating the pulse from a capacitor after discharging it into the sample. In some embodiments, a square wave pulse has a lower voltage at the end of the pulse than at the beginning of the pulse. Square wave pulses can be described by the following formula:

$$\ln (V_o / V_t) = t/ (RC)$$

where the variables are as indicated above. Electroporation devices which can administer square wave pulses are commercially available. For example, the Genepulser Xcell (Bio-Rad, Munich, Germany) can be used.

The improved RNA electroporation methods of the invention can provide a DC population that has been transiently transfected with RNA. Such a population can comprise many cells, for example, at least $1 \times 10^6$ cells, or at least $2 \times 10^6$, $3 \times 10^6$, $4 \times 10^6$, $5 \times 10^6$, $6 \times 10^6$, $7 \times 10^6$, $8 \times 10^6$, or $9 \times 10^6$ cells, or at least $1 \times 10^7$ $2 \times 10^7$ $3 \times 10^7$, $4 \times 10^7$, $5 \times 10^7$, $6 \times 10^7$, $7 \times 10^7$, $8 \times 10^7$, or $9 \times 10^7$ cells, or at least $1 \times 10^8$, $2 \times 10^8$, $3 \times 10^8$ or $4 \times 10^8$ cells. In some embodiments, most of the cells in the population (*i.e.*, at least 60%, 70%, 80%, 90%, or 95% or more) will express at least one peptide encoded by the RNA at sufficiently high levels to result in a phenotypic change in the cell population, such as, for example, the ability to home to lymph nodes *in vivo* or to roll on surfaces coated with sialyl-Lewis[x] *in vitro* where the RNA encodes a selectin. DCs and DC populations of the invention may express more than one peptide that is encoded by the transfected RNA; for example, they may express a membrane homing peptide as well as an antigen of interest that may be presented to T cells. Further, they may express more than one antigen of interest, or a number of antigens of interest.

As used herein, a "membrane homing polypeptide" is a cell surface marker which is capable of interacting with or binding to a ligand present on another surface, such as the surface of a cell or the surface of the extracellular matrix. In some embodiments, a membrane homing polypeptide binds to a ligand present on the surface of a high endothelial venule. A suitable membrane homing polypeptide may also bind to a ligand present on the surface of another type of blood vessel, such as, for example, low-order lymph node venules. Any membrane homing polypeptide may be used in the compositions and methods of the invention so long as its expression contributes to the migration to a lymph node of a DC expressing it. It is understood that a membrane homing polypeptide may be further processed or modified following its translation from the transfected RNA.

In some embodiments, the membrane homing polypeptide is a selectin. As used herein, a "selectin" is a polypeptide that binds to sugar moieties on specific glycoproteins including but not limited to peripheral node addressin ("PNAd"). PNAd refers to a group of glycoproteins and/or glycoconjugates which are present on the surface of lymph node venular endothelium (see, *e.g.*, U.S. Pat. No. 5,538,724). In some embodiments, peripheral node addressin is defined by monoclonal antibody MECA-79, which recognizes a sulfated oligosaccharide carried by the sialomucins; this epitope overlaps with 6-sulfo-sialyl-Lewis[x] (see, *e.g.,* Rosen et al. (2005) Am. J. Pathol. 166: 935-944; Streeter et al. (1988) J. Cell. Biol. 107: 1853). The MECA-79 antibody is commercially available from BD Biosciences Pharmingen (San Diego, CA). Alternatively, PNAd is defined to be a ligand to which L-selectin binds. L-selectin recognizes by selective binding several glycoprotein ligands which are present on the high endothelial venules of the peripheral lymph nodes, including but not limited to GlyCAM-1 (Sgp50), CD34 (Sgp-90), Sgp200, MAdCAM-1, PSGL-1, PCLP, and PCLP-2 (see, *e.g.*, U.S. Pat. Nos. 6,929,792 and 6,395,882).

By "selective binding" is intended that a molecule (such as, for example, L-selectin) binds to a particular ligand as evaluated by ELISA assay more than 5, 7, 9, 10, 20, or more fold above the binding shown to a control ligand, such as, for example, bovine serum albumin (BSA). Alternatively, selective binding by L-selectin may be assayed by using L-selectin-IgG chimera to precipitate inorganic sulfate-labeled material from lymph nodes labeled with $^{35}$S-sulfate in organ culture, as described, for example, in U.S. Pat. No. 5,484,891. By "binding" as used herein is intended that molecules may be covalently or non-covalently bound to each other.

Thus, the term "selectin" encompasses various forms of native selectin such as L-selectin, E-selectin, and P-selectin as well as modified forms of selectin, including those which have been engineered to alter or remove the protease cleavage sites present in the native selectins. The term "selectin" also encompasses functional chimeric proteins such as the E/L selectin chimera described in U.S. Pat. No. 6,929,792, and also includes chimeric proteins which combine portions or domains from different native selectins such as, for example, a chimeric molecule comprising the extracellular domain of native human E-selectin and the transmembrane and intracellular domains of native human L-selectin ("E/L-selectin") (see, *e.g.*, Robert et al. (2003) Gene Ther. 10: 1479-1486). Other domains of native selectins are known in the art and can be combined into chimeric proteins (see, *e.g.*, Smalley and Ley (2005) J. Cell. Mol. Med. 9: 255-266). As used herein, a "chimera" or "chimeric protein" combines at least one domain from one protein with at least one domain from another protein. The proteins may be native or they may be modified.

As used in herein, the singular form "a," "an," and "the" include plural references unless the context clearly dictates otherwise. For example, the term "a cell" includes a plurality of cells, including mixtures thereof. All numerical designations, *e.g.*, pH, temperature, time, concentration, and molecular weight, including ranges, are approximations which are varied (+) or (-) by increments of 0.1, unless otherwise explicitly stated.

As used herein, the term "comprising" is intended to mean that the compositions and methods include the recited elements, but not excluding others. "Consisting essentially of" when used to define compositions and methods indicates the exclusion of other elements of any essential significance to the combination. Thus, a composition consisting essentially of the elements as defined herein would not exclude trace contaminants from the isolation and purification method and would also not exclude pharmaceutically acceptable carriers or other commonly-used additives, such as phosphate buffered saline, preservatives, and the like. "Consisting of" indicates the exclusion of more than trace elements of other ingredients or, for methods, the exclusion of substantial method steps other than those explicitly mentioned. Embodiments defined by each of these transition terms are within the scope of this invention.

As used herein, "isolated" refers to cells that are separated from their native environment and present in quantities sufficient to permit identification and use of the cells. As used herein with respect to dendritic cells, it means removed from the source of the DCs, *i.e.,* the anatomical site in which they are found in an organism, *e.g.*, blood or the like. DC cell cultures can be essentially pure but need not be pure for use herein, *e.g.*, in a vaccine.

"Immune response" broadly refers to the antigen-specific responses of lymphocytes to any substance. Any substance that can elicit an immune response is said to be "immunogenic" and is referred to as an "immunogen." All immunogens are antigens, but not all antigens are immunogenic. An immune response of this invention can be humoral (via antibody activity) or cell-mediated (via T-cell activation).

The term "major histocompatibility complex" or "MHC" refers to a complex of genes encoding cell-surface molecules that are required for antigen presentation to T cells and for rapid graft rejection. In humans, the MHC is also known as the "human leukocyte antigen" or "HLA" complex. The proteins encoded by the MHC are known as "MHC molecules" and are classified into Class I and Class II MHC molecules. Class I MHC molecules include membrane heterodimeric proteins made up of an $\alpha$ chain encoded in the MHC noncovalently linked with the $\beta_2$-microglobulin. Class I MHC molecules are expressed by nearly all nucleated cells and have been shown to function in antigen presentation to CD8$^+$ T cells. Class I molecules include HLA-A, B, and C in humans. Class II MHC molecules also include membrane heterodimeric proteins consisting of noncovalently associated $\alpha$ and $\beta$ chains. Class II MHC molecules are known to function in antigen-presentation to CD4$^+$ T cells and, in humans, include HLA-DP, -DQ, and -DR.

The term "antigen presenting cells" (APCs) refers to a class of cells capable of presenting one or more antigens in the form of peptide-MHC complex recognizable by specific effector cells of the immune system, thereby inducing an effective cellular immune response against the antigen(s) being presented. APCs can be intact whole cells such as macrophages, B-cells, endothelial cells, activated T-cells, and dendritic cells; or other molecules, naturally occurring or synthetic, such as purified MHC Class I molecules complexed to β2-microglobulin. While many types of cells may be capable of presenting antigens on their cell surface for T-cell recognition, only dendritic cells have the capacity to present antigens in a context suitable to activate naive T-cells for cytotoxic T-lymphocyte (CTL) responses, *e.g.*, in combination with proper co-stimulatory molecules.

The term "immune effector cells" refers to cells capable of binding an antigen and which mediate an immune response. These cells include, but are not limited to, T cells, B cells, monocytes, macrophages, NK cells and cytotoxic T lymphocytes (CTLs), for example CTL lines, CTL clones, and CTLs from tumor, inflammatory, or other infiltrates. A "naïve" immune effector cell is an immune effector cell that has never been exposed to an antigen capable of activating that cell. Activation of naive immune effector cells requires both recognition of the peptide:MHC complex and the simultaneous delivery of a costimulatory signal by a professional APC in order to proliferate and differentiate into antigen-specific, armed effector T cells.

As used herein, the term "educated" antigen-specific immune effector cell is an immune effector cell which has previously encountered an antigen. In contrast to its naive counterpart, the activation of an educated, antigen-specific immune effector cell does not require a costimulatory signal. Recognition of the peptide:MHC complex is sufficient. The term "activated" when used in reference to a T cell implies that the cell is no longer in G0 phase, and begins to produce one or more of cytotoxins, cytokines and other related membrane-associated proteins characteristic of the cell type (*e.g.*, CD8+ or CD4+), and is capable of recognizing and binding any target cell that displays the particular peptide/MHC complex on its surface, and releasing its effector molecules. "Co-stimulatory molecules" are involved in the interaction between receptor-ligand pairs expressed on the surface of antigen presenting cells and T cells. Research accumulated over the past several years has demonstrated convincingly that resting T cells require at least two signals for induction of cytokine gene expression and proliferation (see, *e.g.*, Schwartz (1990) Science 248: 1349-1356 and Jenkins (1992) Immunol. Today 13: 69-73). One signal, the one that confers specificity, can be produced by interaction of the TCR/CD3 complex with an appropriate MHC/peptide complex. The second signal is not antigen specific and is termed the "co-stimulatory" signal. This signal was originally defined as an activity provided by bone-marrow-derived accessory cells such as macrophages and dendritic cells, the so called "professional" APCs.

Several molecules have been shown to provide and/or enhance co-stimulatory activity. These include heat stable antigen (HSA) (Liu et al. (1992) J. Exp. Med. 175: 437-445), chondroitin sulfate-modified MHC invariant chain (li-CS) (Naujokas et al. (1993) Cell 74: 257-268), intracellular adhesion molecule 1 (ICAM-1) (Van Seventer (1990) J. Immunol. 144: 4579-4586), B7.1/CD80, and B7.2B70/CD86 (Schwartz (1992) Cell 71: 1065-1068). These molecules each appear to provide and/or assist co-stimulation by interacting with their cognate ligands on the T cells. Co-stimulatory molecules mediate co-stimulatory signal(s), which are necessary, under normal physiological conditions, to achieve full activation of naive T cells. One exemplary receptor-ligand pair is the B7 family of co-stimulatory molecule on the surface of APCs and its counter-receptor CD28 or CTLA-4 on T cells (Freeman et al. (1993) Science 262: 909-911; Young, et al. (1992) J. Clin. Invest. 90: 229 and Nabavi et al. (1992) Nature 360: 266-268). Other important co-stimulatory molecules are CD40 and CD54.

The term "co-stimulatory molecule" encompasses any single molecule or combination of molecules which, when acting together with a MHC/peptide complex bound by a TCR on the surface of a T cell, provides a co-stimulatory effect which achieves activation of the T cell that binds the peptide. The term thus encompasses B7 or other co-stimulatory molecule(s) on an antigen-presenting matrix such as an APC, fragments thereof (alone, complexed with another molecule(s), or as part of a fusion protein) which, together with MHC complex, binds to a cognate ligand and results in activation of the T cell when the TCR on the surface of the T cell specifically binds the peptide. The term "co-stimulatory molecule" also refers to molecules having similar biological activity as wild-type or purified co-stimulatory molecules (*e.g.*, recombinantly produced or muteins thereof).

As used herein, the term "cytokine" refers to any one of the numerous soluble factors that exert a variety of effects on cells, for example, inducing growth or proliferation. Non-limiting examples of cytokines which may be used alone or in combination in the practice of the present invention include interleukin-2 (IL-2), stem cell factor (SCF), interleukin-3 (IL-3), interleukin-6 (IL-6), interleukin-12 (IL-12), G-CSF, granulocyte macrophage-colony stimulating factor (GM-CSF), interleukin-1 alpha (IL-1$\alpha$), interleukin-1L (IL-11), MIP-11, leukemia inhibitory factor (LIF), c-kit ligand, thrombopoietin (TPO) and flt3 ligand. Cytokines are commercially available from several vendors such as, for example, Genzyme (Framingham, MA), Genentech (South San Francisco, CA), Amgen (Thousand Oaks, CA), R&D Systems (Minneapolis, MN) and Immunex (Seattle, WA). The term "cytokine" also encompasses molecules having similar biological activity as wild-type or purified cytokines (*e.g.*, recombinantly produced or muteins thereof).

The terms "polynucleotide," "nucleic acid," and "nucleic acid molecule" are used interchangeably to refer to polymeric forms of nucleotides of any length. The polynucleotides may contain deoxyribonucleotides, ribonucleotides, and/or their analogs. Nucleotides may have any three-dimensional structure, and may perform any function, known or unknown. The term "polynucleotide" includes, for example, single-stranded, double-stranded and triple helical molecules, a gene or gene fragment, exons, introns, mRNA, tRNA, rRNA, ribozymes, cDNA, recombinant polynucleotides, branched polynucleotides, plasmids, vectors, isolated DNA of any sequence, isolated RNA of any sequence, nucleic acid probes, and primers. In addition to a native nucleic acid molecule, a nucleic acid molecule of the present invention may also comprise modified nucleic acid molecules. As used herein, mRNA refers to an RNA that can be translated in a dendritic cell. Such mRNAs typically are capped, have a ribosome binding site (Kozak sequence) and a translational initiation codon preceding an open reading frame coding for a peptide or protein of choice, and usually contain a poly A-tail.

The term "peptide" is used in its broadest sense to refer to a compound of two or more subunit amino acids, amino acid analogs, or peptidomimetics. The subunits may be linked by peptide bonds. In another embodiment, the subunit may be linked by other bonds, *e.g.*, ester, ether, *etc.* As used herein, the term "amino acid" refers to either natural and/or unnatural or synthetic amino acids, including glycine and both the D- and L-optical isomers, amino acid analogs and peptidomimetics. A peptide of three or more amino acids is commonly called an oligopeptide if the peptide chain is short. If the peptide chain is long, the peptide is commonly called a polypeptide or a protein.

Polypeptides and proteins for use in the methods of the invention can be obtained by chemical synthesis using a commercially available automated peptide synthesizer such as those manufactured by Perkin Elmer/Applied Biosystems, Inc., Model 430A or 431A, Foster City, CA, USA. The synthesized protein or polypeptide can be precipitated and further purified, for example by high performance liquid chromatography (HPLC). Alternatively, the proteins and polypeptides can be obtained using known recombinant methods.

As used herein, "expression" refers to the processes by which polynucleotides are transcribed into mRNA; the mRNA may further be translated into peptides, polypeptides, or proteins. Thus, as used herein, the term "expression" encompasses both transcription and translation, unless otherwise indicated. Regulatory elements required for expression of both mRNA and peptides, polypeptides, or proteins are known in the art. "Under transcriptional control" is a term understood in the art and indicates that transcription of a polynucleotide sequence, usually a DNA sequence, depends on its being operatively linked to an element which contributes to the initiation of, or promotes, transcription. "Operatively linked" indicates that elements are in an arrangement allowing them to function and/or to exert their effects on another element. For example, a transcriptional enhancer may be operatively linked to a promoter when both are included on the same expression vector.

Vectors that contain versatile cloning sites and necessary elements for expression are known in the art. Depending on the circumstances, vectors with various features may be used, and one of skill in the art is familiar with the various features and the circumstances in which they are appropriate. Vectors may contain, for example, some or all of the following: a selectable marker gene for selection of stable or transient transfectants in mammalian cells (*e.g.*, neomycin resistance); enhancer and/or promoter sequences for high levels of transcription (*e.g.*, from the immediate early gene of human CMV); transcription termination and RNA processing signals for mRNA stability (*e.g.*, from SV40); SV40 polyoma origins of replication and ColEl for proper episomal replication; versatile multiple cloning sites; and promoters for in *vitro* transcription of sense and/or antisense RNA (*e.g.*, the T7 and/or SP6 promoters). Other examples of these and other useful elements are known and available in the art. For example, some useful vectors are capable of transcribing RNA *in vitro* or *in vivo,* and are commercially available from sources such as Stratagene (La Jolla, CA) and Promega Biotech (Madison, WI). In some embodiments, expression vectors are used to produce RNA which is then used to transfect a cell population.

In order to optimize expression and/or *in vitro* transcription, certain modifications known in the art may be necessary or helpful. In some embodiments, *in vitro* transcribed mRNA is optimized for stability and efficiency of translation. For example, mRNA stability and/or translational efficiency can be increased by including 3' UTRs and/or 5' UTRs in the mRNA. Preferred examples of 3' UTRs include those from human CD40, β-actin and rotavirus gene 6. Preferred examples of 5' UTRs include the one from CD40L, and the translational enhancers in the 5' UTRs of Hsp70, VEGF, spleen necrosis virus RU5, and tobacco etch virus. Other examples of regulatory elements include, but are not limited to, the following.

Beta-actin is an abundantly expressed gene in human non-muscle cells. The human β-actin promoter has been widely used to drive gene expression in mammalian cell lines and transgenic mice. In constructs containing this promoter, inclusion of the β-actin 3' UTR plus flanking region has been demonstrated to further increase the level of mRNA accumulation (see, *e.g.,* Qin and Gunning (1997) J. Biochem. Biophys. Meth. 36: 63-72).

The 3' UTR of the simian rotavirus gene 6 mRNA functions as an enhancer of translation in its capped, non-polyadenylated viral transcript. The 3' UTR has also been shown to enhance translation of a heterologous reporter mRNA in rabbit reticulocyte lysates (see, *e.g.,* Yang et. al. (2004) Arch. Virol 149: 303-321).

The 5' UTR of the human hsp70 gene has been shown to increase translation of reporter mRNAs in the absence of stress induction and without dramatically influencing the message stability. Enhancer function has been demonstrated in a number of human cell lines (see, e.g., Vivinus, et al. (2001) Eur. J. Biochem. 268: 1908-1917).

The mouse VEGF 5' UTR enhances translation of a monocistronic reporter RNA and also has IRES (Internal Ribosome Entry Site) activity. Its enhancer activity has been demonstrated in rat, hamster and human cell lines. The full length 5'UTR is 1014 nucleotides, but a 163 nucleotide mutant version was shown to be more active (see, *e.g.,* Stein et al. (1998) Mol. Cell. Biol. 18: 3112-3119).

The Spleen Necrosis Virus (SNV) is an avian retrovirus. The RU5 region of the viral 5' LTR stimulates translation efficiency of a non-viral reporter RNA in human 293 cells (see, *e.g.,* Roberts and Boris-Lawrie (2000) J. Virol. 74: 8111-8118).

The 143-nucleotide 5' leader of the tobacco etch virus RNA promotes cap-independent translation of reporter mRNAs in plant and animal cell lines. Although the leader sequence does not further enhance the translation of capped transcripts, the cap-independent CD40L expression in dendritic cells is a very attractive alternative to *in vitro* capping (see, *e.g.,* Gallie et al. (1995) Gene 165: 233-238; Niepel and Gallie (1999) J. Virol. 73: 9080-9088; Gallie (2001) J. Virol. 75: 12141-12152).

"Gene delivery" or "gene transfer" as used herein refers to the introduction of an exogenous polynucleotide into a host cell, irrespective of the method used for the introduction. Gene delivery (or transfer) refers to the delivery of a nucleic acid that may be integrated into the host cell's genome, or that may replicate independently of the host cell genome. Gene delivery does not refer to introduction of an mRNA into a cell. A "gene delivery vehicle" is any molecule that can carry inserted polynucleotides into a host cell. Examples of gene delivery vehicles include, for example: liposomes; biocompatible polymers (natural or synthetic); lipoproteins; polypeptides; polysaccharides; lipopolysaccharides; artificial viral envelopes; and metal particles. Examples of gene delivery vehicles also include bacteria or viruses (such as, for example, baculovirus, adenovirus and retrovirus), bacteriophage, vectors (*e.g.*, cosmids and plasmids), and other recombination vehicles known in the art which have been described for expression in a variety of eukaryotic and prokaryotic hosts, and may be used for gene therapy as well as for the production of polynucleotides or proteins.

A number of vectors are capable of mediating transfer of genes to mammalian cells, as is known in the art and described herein. A "viral vector" is defined as a recombinantly produced virus or viral particle that comprises a polynucleotide to be delivered into a host cell, either *in vivo, ex vivo* or *in vitro.* Examples of viral vectors include retroviral vectors, adenoviral vectors, adeno-associated viral vectors, alphaviral vectors and the like. Alphaviral vectors, such as Semliki Forest virus-based vectors and Sindbis virus-based vectors, have also been developed for use in gene therapy and immunotherapy. See, *e.g.*, Schlesinger and Dubensky (1999) Curr. Opin. Biotechnol. 5:434-439 and Zaks et al. (1999) Nat. Med. 7: 823-827.

In aspects where gene transfer is mediated by a retroviral vector, a vector construct refers to the polynucleotide comprising the retroviral genome or part thereof, and a therapeutic gene. As used herein, the terms "retroviral mediated gene transfer" and "retroviral transduction" have the same meaning and refer to the process by which a gene or nucleic acid sequences are stably transferred into the host cell by virtue of the virus entering the cell and integrating its genome into the host cell genome. The virus can enter the host cell via its normal mechanism of infection or be modified such that it binds to a different host cell surface receptor or ligand to enter the cell. As used herein, "retroviral vector" refers to a viral particle capable of introducing exogenous nucleic acid into a cell through a viral or viral-like entry mechanism. Retroviruses carry their genetic information in the form of RNA; however, once the virus infects a cell, the RNA is reverse-transcribed into DNA which integrates into the genomic DNA of the infected cell and is called a provirus.

Two or more polynucleotides or polynucleotide regions (or polypeptides or polypeptide regions) may share a certain percentage of "sequence identity." Alignment and evaluation of the sequence identity shared by nucleotide or amino acid sequences is determined using the BLAST alignment program with the default parameters. The BLAST program is an implementation of the algorithm of Karlin and Altschul (1990) Proc. Natl. Acad. Sci. USA 87: 2264, modified as in Karlin and Altschul (1993) Proc. Natl. Acad. Sci. USA 90: 5873-5877. Alternative programs are BLASTN and BLASTP, using the following default parameters: genetic code = standard; filter = none; strand = both; cutoff = 60; expect = 10; matrix = BLOSLTM62; descriptions = 50 sequences; sort by = HIGH SCORE; databases = non-redundant, GenBank + EMBL + DDBJ + PDB + GenBank CDS translations + SwissProtein + SPupdate + PIR. Sequences may also be compared using the GAP program, which uses the algorithm of Needleman and Wunsch ((1970) J. Mol. Biol. 48:443-453) to find the alignment of two complete sequences that maximizes the number of matches and minimizes the number of gaps. Details of these programs can be found at the URL ncbi.nlm.nih.gov/cgi-bin/BLAST. Sequence comparison software, including the BLAST and GAP programs, is commercially available as the Accelrys GCG package (version 11.0, Accelrys, San Diego). Sequences may share varying percentages of sequence identity, such as, for example, at least 75%, 80%, 85%, 90%, 95%, 96%, 97%, 98%, 99% or more sequence identity. Alignments and sequence identity evaluations may also be performed manually using the algorithms indicated above, or another algorithm.

A "conservative alteration" of an amino acid sequence or a nucleotide sequence encoding it is one that results in an alternative amino acid sequence of similar charge density, hydrophilicity or hydrophobicity, size, and/or configuration (*e.g.*, Val for Ile). In comparison, a "nonconservative alteration" is one that results in an alternative amino acid sequence of significantly different charge density, hydrophilicity or hydrophobicity, size and/or configuration (*e.g.*, Val for Phe). The means of making such modifications are well-known in the art and also can be accomplished by means of commercially available kits and vectors (for example, those available from New England Biolabs, Inc., Beverly, Mass.; Clontech, Palo Alto, Calif.).

An "isolated" or "purified" nucleic acid molecule, polynucleotide, peptide, polypeptide, protein, antibody, or fragments thereof is substantially or essentially free from components that normally accompany or interact with the nucleic acid molecule or protein as found in its naturally occurring environment. Thus, an isolated or purified nucleic acid molecule or protein is substantially free of other cellular material, or culture medium when produced by recombinant techniques, or substantially free of chemical precursors or other chemicals when chemically synthesized. For example, preferably, an "isolated" polynucleotide is free of sequences (such as protein encoding sequences) that naturally flank the polynucleotide in the genomic DNA of the organism from which the polynucleotide is derived (*i.e.*, sequences located at the 5' and 3' ends of the nucleic acid). For example, in various embodiments, an isolated polynucleotide can contain less than about 5 kb, 4 kb, 3 kb, 2 kb, 1 kb, 0.5 kb, or 0.1 kb of nucleotide sequences that naturally flank the polynucleotide in genomic DNA. A protein that is substantially free of cellular material includes preparations of protein having less than about 30%, 20%, 10%, 5%, or 1% (by dry weight) of contaminating protein. When the protein of the invention or biologically active portion thereof is recombinantly produced, preferably culture medium represents less than about 30%, 20%, 10%, 5%, or 1% (by dry weight) of chemical precursors or chemicals other than the protein of interest.

Polynucleotides can be replicated by any method known in the art. PCR technology is one means to replicate DNA and is described in United States Patent Nos. 4,683,195; 4,800,159; 4,754,065; and 4,683,202; and is also described in PCR: THE POLYMERASE CHAIN REACTION (Mullis et al. eds, Birkhauser Press, Boston (1994)) and references cited therein. Additional methods to generate polynucleotides are known in the art.

A "concentrated" polynucleotide, peptide, polypeptide, protein, antibody, or fragment(s) thereof is distinguishable from a naturally occurring counterpart in that the concentration or number of molecules per volume is greater than that of its naturally occurring counterpart. As is apparent to those of skill in the art, a non-naturally occurring polynucleotide, peptide, polypeptide, protein, antibody, or fragment(s) thereof, does not require "isolation" to distinguish it from a naturally occurring counterpart because it is distinguishable from its naturally occurring counterpart, for example, by its primary sequence, or alternatively, by another characteristic such as its glycosylation pattern, or by some other characteristic.

The terms "host cell," "target cell," and "recipient cell" are intended to include any individual cell or cell culture which can be or have been recipients for vectors or the incorporation of exogenous nucleic acid molecules, polynucleotides and/or proteins. It also is intended to include progeny of a single cell, and the progeny may not necessarily be completely identical (in morphology or in genomic or total DNA complement) to the original parent cell due to natural, accidental, or deliberate mutation. The cells may be prokaryotic or eukaryotic, and include but are not limited to bacterial cells, yeast cells, animal cells, and mammalian cells, *e.g.*, murine, rat, simian or human cells.

A "subject" is a vertebrate, such as for example, a mammal or a human. Mammals include, but are not limited to, murines, simians, humans, farm animals, sport animals, and pets. A "patient" is a subject in need of treatment, for example, for a particular disease or disorder, such as cancer. However, the exact disease or disorder need not be identified in order for a patient to be treated. A "control" is an alternative subject or sample used in an experiment for comparison. A control can be positive or negative. For example, where the purpose of the experiment is to determine a correlation of an immune response with a particular culture condition, it is generally preferable to use a positive control and a negative control. Criteria and parameters for appropriate controls are known in the art and can be readily determined by one of skill in the art. For example, a suitable control for a transfected dendritic cell under some circumstances is a dendritic cell of the same or similar lineage which has been "mock transfected," *e.g.*, which has been electroporated without RNA. Alternatively, a suitable control for a transfected dendritic cell under some circumstances is a dendritic cell which has been transfected with RNA encoding a different protein that is not expected to affect the phenotype of interest.

By "cancer" is meant at least one abnormal cell which exhibits relatively autonomous growth. A cancer cell exhibits an aberrant growth phenotype characterized by a significant loss of cell proliferation control. Cancerous cells can be benign or malignant. Cancer can affects cells of various tissues or organs, including, for example, the bladder, blood, brain, breast, colon, digestive tract, lung, ovaries, pancreas, prostate gland, or skin. The definition of a cancer cell, as used herein, includes not only a primary cancer cell, but also any cell derived from a cancer cell ancestor. This includes metastasized cancer cells, and *in vitro* cultures and cell lines derived from cancer cells.

Cancer includes, but is not limited to, solid tumors, liquid tumors, hematologic malignancies, renal cell cancer, melanoma, breast cancer, prostate cancer, testicular cancer, bladder cancer, ovarian cancer, cervical cancer, stomach cancer, esophageal cancer, pancreatic cancer, lung cancer, neuroblastoma, glioblastoma, retinoblastoma, leukemias, myelomas, lymphomas, hepatomas, adenomas, sarcomas, carcinomas, blastomas, *etc.* When referring to a type of cancer that normally manifests as a solid tumor, a "clinically detectable" tumor is one that is detectable on the basis of tumor mass, *e.g.*, by such procedures as CAT scan, magnetic resonance imaging (MRI), X-ray, ultrasound or palpation. Biochemical or immunologic findings alone may be insufficient to meet this definition.

The term "culturing" refers to the *in vitro* maintenance, differentiation, and/or propagation of cells or in suitable media. By "enriched" is meant a composition comprising cells present in a greater percentage of total cells than is found in the tissues where they are present in an organism. For example, the enriched cultures and preparations of DCs made by the methods of the invention are present in a higher percentage of total cells as compared to their percentage in the tissues where they are present in an organism (*e.g.*, blood, skin, lymph nodes, *etc.).

In some embodiments, a "composition" is intended to encompass a combination of active agent and another compound or composition, either inert (for example, a detectable agent or label) or active (for example, an adjuvant). A "pharmaceutical composition" or medicament is intended to include the combination of an active agent with a carrier, inert or active, making the composition suitable for diagnostic or therapeutic use *in vitro, in vivo* or *ex vivo.* As used herein, the term "pharmaceutically acceptable carrier" encompasses any of the standard pharmaceutical carriers, such as a phosphate buffered saline solution, water, and emulsions, such as an oil/water or water/oil emulsion, and various types of wetting agents. The compositions also can include stabilizers and preservatives. For examples of carriers, stabilizers and adjuvants, see Martin REMINGTON'S PHARM. SCL, 18th Ed. (Mack Publ. Co., Easton (1990)).

An "effective amount" is an amount sufficient to effect at least one beneficial or desired result, such as, for example, enhanced immune response or amelioration of a medical condition (disease, infection, *etc.).* An effective amount can be administered in one or more administrations, applications or dosages. Suitable dosages will vary depending on body weight, age, health, disease or condition to be treated and route of administration.

In some embodiments, the steps of the method can be performed *in vivo* or *ex vivo.* When practiced *ex vivo,* the method can be practiced in an open or closed system. Methods and systems for culturing and enriching cell populations are known in the art. See, examples 1 and 2 of U.S. Patent Publication No. 2004/0072347. See also U.S. Patent Publication No. 2003/0235908, which describes closed systems for cell expansion.

In some embodiments of the invention, dendritic cells may additionally be loaded with one or more antigens of interest which will be then be processed and/or presented by the mature DCs. DCs may be loaded when they are immature or when they are mature, or a precursor cell may be loaded and then used to generate a mature, loaded DC. By "loaded" or "antigen-loaded" is meant that antigen is incorporated into a dendritic cell such that it can be presented by the dendritic cell to a T cell. In some embodiments, the dendritic cells are loaded by being cultured in medium containing the antigen (sometimes referred to as "pulsing" or "pulsed"; see, *e.g.*, Shaw et al. (2002) Infect. Immun. 70: 1097-1105). The DCs then take up and process the antigen on the cell surface in association with MHC molecules. In other embodiments, the DCs are loaded with antigen by transfection with a nucleic acid encoding the antigen, *e.g.*, the DCs are electroporated with RNA encoding the antigen, which they then express.

The term "antigen" is well understood in the art and includes substances which are immunogenic, *i.e.,* an immunogen. It will be appreciated that the use of any antigen is envisioned for use in the present invention and thus the term "antigen" includes but is not limited to a self-antigen (whether normal or disease-related), an infectious or pathogen-specific antigen (*e.g.*, a microbial antigen, viral antigen, *etc.),* or some other foreign antigen (*e.g.*, a food component, pollen, *etc.).* For example, antigens include, but are not limited to, pathogens, pathogen lysates, pathogen extracts, pathogen polypeptides, viral particles, bacteria, proteins, polypeptides, cancer cells, cancer cell lysates, cancer cell extracts, and cancer-cell-specific polypeptides. Antigens may also act to enhance viability or immunogenicity, as is known in the art. A "pathogen-specific" antigen is an antigen which is produced by a particular pathogen or a particular group of pathogens, but not by another pathogen or group of pathogens, respectively. Examples of pathogen-specific antigens are known in the art, including HIV-specific antigens (see, *e.g.*, Stratov et al. (2004) Curr. Drug Targ. 5: 71-88) and HCV-specific antigens (see, *e.g.*, Simon et al. (2003) Infect. Immun. 71: 6372-6380; Encke et al. (1998) J. Immunol. 161: 4917-4923).

Antigens can be naturally occurring or recombinantly produced. The term "antigen" applies to collections of more than one antigen, so that immune responses to multiple antigens may be modulated simultaneously. Moreover, the term includes any of a variety of different formulations of antigen. An "epitope" is the portion of an antigen (usually a surface portion or an MHC-binding peptide) which elicits the immune response and is generally encompassed by the term "antigen" as used herein.

The antigens can be delivered to the cells as polypeptides or proteins, or they can be delivered as nucleic acid molecules or polynucleotides encoding the antigens, so that expression of the nucleic acid molecules or polynucleotides results in antigen production either in the individual being treated (when delivered *in vivo*) or the cell culture system (when delivered *in vitro*)*.* Thus, the methods of the invention further comprise introducing into immature or mature DCs one or more antigens or a polynucleotide(s) encoding one or more antigens to produce antigen-loaded DCs by any suitable method (*e.g.*, transfection, pulsing, and the like). In some embodiments, mRNA encoding the antigen(s) is introduced into the cells by electroporation. In some embodiments, this mRNA can be introduced together with an mRNA encoding a CD40 agonist or substantially concurrent with CD40 agonist signaling.

The antigen may be delivered in its "natural" form, *i.e.*, where no human intervention was involved in preparing the antigen or inducing it to enter the environment in which it encounters the DC. Alternatively or additionally, the antigen may be delivered in a crude preparation, for example, of the type that is commonly administered in a conventional allergy shot or in a tumor lysate. The antigen may alternatively be substantially purified, *e.g.*, at least about 90% pure. In some embodiments, the antigen is delivered to the antigen presenting cell in the form of RNA isolated or derived from a neoplastic cell or a pathogen or a pathogen-infected cell, *i.e.*, in bulk. Methods for RT-PCR of RNA extracted from any cell and *in vitro* transcription are disclosed, for example, in PCT/US05/053271.

Where the antigen is a peptide, it may be generated, for example, by proteolytic cleavage of isolated proteins. Any of a variety of cleavage agents may be utilized, including, but not limited to: pepsin, cyanogen bromide, trypsin, chymotrypsin, *etc.* Alternatively, peptides may be chemically synthesized, preferably on an automated synthesizer (see, for example, Stewart et al., Solid Phase Peptide Synthesis, 2d. Ed., Pierce Chemical Co., 1984). In some embodiments, recombinant techniques may be employed to create a nucleic acid encoding the peptide of interest, and to express that peptide under desired conditions (*e.g.*, in a host cell or an *in vitro* expression system from which it can readily be purified).

In some embodiments, the antigen can have a structure that is distinct from any naturally-occurring compound. In certain embodiments of the invention, the antigen is a "modified antigen" having a structure that is substantially similar to that of a naturally-occurring antigen but that includes one or more deviations from the precise structure of the naturally-occurring compound. For instance, where the naturally-occurring antigen is a protein or polypeptide antigen, a modified antigen as compared with that protein or polypeptide antigen would have an amino acid sequence that differs from that of the naturally-occurring antigen in the addition, substitution, or deletion of one or more amino acids, and/or would include one or more amino acids that differ from the corresponding amino acid in the naturally-occurring antigen by the addition, substitution, or deletion of one or more chemical moieties covalently linked to the amino acid. In one aspect, the naturally-occurring and modified antigens share at least one region of at least 5 amino acids that are at least approximately 75% identical. Those of ordinary skill in the art will appreciate that, in comparing two amino acid sequences to determine the extent of their identity, the spacing between stretches (*i.e.*, regions of at least two) of identical amino acids need not always be precisely preserved. Naturally-occurring and modified protein or polypeptide antigens can show at least approximately 80% identity, more alternatively 85%, 90%, 95%, or greater than 99% identity in amino acid sequence for at least one region of at least 5 amino acids. Often, it may be useful for a much longer region of amino acid sequence to show the designated degree of identity (*e.g.*, a region comprising 10, 20, 50, or 100 or more amino acids).

In some embodiments, the DCs express at least one other antigen from a cancer cell or a pathogen, such as, for example, HIV or HCV. The term "tumor associated antigen" or "TAA" refers to an antigen that is associated with a cancer. Examples of particular tumor-associated antigens that can be expressed by DCs of the invention to induce or enhance immune response to cancerous cells are known in the art and include, for example, MAGE proteins, MART, LAGE, NY-ESO-1, tyrosinase, PRAME, prostate specific antigen (PSA), Melan-A, and others (see, *e.g.*, Santin et al. (2005) Curr. Pharm. Des. 11: 3485-3500; Rimoldi et al. (2000) J. Immunol. 165: 7253-7261; Watari et al. (2000) FEBS Lett. 466: 367-371; Engelhard et al. (2000) Cancer J. 6 Suppl. 3: S272-S280; Chakraborty et al. (2003) Cancer Immunol. Immunother. 52: 497-502; Romero et al. (2002) Immunol. Rev. 188: 81-96). The use of an antigen of interest which is a tumor-associated antigen is useful for modulation of a subject's immune response for treatment of cancer. Thus, in some embodiments, the invention provides methods for treating cancer.

The invention provides methods for delivering antigen-loaded dendritic cells to lymph tissue in a patient, for example, comprising the steps of: (a) providing isolated dendritic cells which have been transiently transfected with RNA encoding a membrane homing polypeptide and which have been loaded with at least one other antigen; and (b) intravenously administering the dendritic cells to the patient. In some embodiments, the other antigen is defined and in some embodiments it is not defined. An antigen is "defined" when the identity and length of each of the peptides or proteins of the antigen is known in advance. Conversely, an antigen is "undefined" or "not defined" where the identity and/or length of at least one peptide or protein component of the antigen is not known in advance. For example, an antigen comprising total cell protein (*e.g.*, an antigen provided to a cell as total cell mRNA) would be an undefined antigen.

The antigen-loaded DCs of the invention are useful for inducing or enhancing an immune response in a subject to the antigen of interest. Thus, in some embodiments, the invention provides a method of inducing or enhancing an immune response in a subject comprising administering to the subject an effective amount of antigen-loaded DCs of the invention. The DCs can be allogeneic or autologous to the subject. An "effective amount" of DCs is sufficient to achieve a desired beneficial therapeutic response in the subject over time, or to inhibit growth of cancer cells, or to inhibit infection.

As used herein, the term "inducing" or "enhancing" an immune response in a subject is a term understood in the art and refers to an increase of at least about 2-fold, 5-fold, 10-fold, 100-fold, 500-fold, or at least about 1000-fold or more in an immune response to an antigen which can be detected or measured, after introducing the antigen into the subject, relative to the immune response (if any) before introduction of the antigen into the subject. An immune response to an antigen includes but is not limited to production of an antigen-specific antibody, and production of an immune cell expressing on its surface a molecule which specifically binds to an antigen.

Methods of determining whether an immune response to a given antigen has been induced (or enhanced) are well known in the art. For example, antigen-specific antibody can be detected using any of a variety of immunoassays known in the art, including, but not limited to, ELISA, wherein, for example, binding of an antibody in a sample to an immobilized antigen is detected with a detectably-labeled second antibody (*e.g.*, enzyme-labeled mouse anti-human Ig antibody).

The dendritic cells of the invention can be provided in a formulation which is suitable for administration to a patient, e.g., intravenously. DCs of the invention that are suitable for administration to a patient are referred to herein as a "vaccine" or "DC vaccine." A vaccine or DC vaccine may further comprise additional components to help modulate the immune response, or it may be further processed in order to be suitable for administration to a patient. Methods of intravenous administration of dendritic cells are known in the art, and one of skill in the art will be able to vary the parameters of intravenous administration in order to maximize the therapeutic effect of the administered DCs.

Thus, DCs are administered to a subject in any suitable manner, often with at least one pharmaceutically acceptable carrier. The suitability of a pharmaceutically acceptable carrier is determined in part by the particular composition being administered, as well as by the particular method used to administer the composition. Most typically, quality control tests (*e.g.*, microbiological assays clonogenic assays, viability tests), are performed and the cells are reinfused back to the subject, in some cases preceded by the administration of diphenhydramine and hydrocortisone. See, *e.g.*, Korbling et al. (1986) Blood 67: 529-532 and Haas et al. (1990) Exp. Hematol. 18: 94-98.

Formulations suitable for parenteral administration, such as, for example, by intravenous administration, include aqueous isotonic sterile injection solutions which can contain antioxidants, buffers, bacteriostats, and solutes that render the formulation isotonic with the blood of the intended recipient, as well as aqueous and non-aqueous sterile suspensions that can include suspending agents, solubilizers, thickening agents, stabilizers, and preservatives.

Generally, DCs of the invention can be administered to a subject at a rate determined by the effective dose, the toxicity of the cell type (*e.g.*, the LD-50), and the side-effects of the cell type at various concentrations, as appropriate to the mass and overall health of the subject as determined by one of skill in the art. Administration can be accomplished via single or divided doses. The DCs of the invention can supplement other treatments for a disease or disorder, including, for example, conventional radiation therapy, cytotoxic agents, nucleotide analogues and biologic response modifiers.

For the purpose of illustration only, DCs of the invention could be administered to a subject as follows. Blood samples are obtained from the subject prior to infusion, and some aliquots are saved for subsequent analysis and comparison. Generally at least about $10^4$ to $10^6$ and typically between $1 \times 10^7$ and $1 \times 10^9$ cells are infused intravenously into a 70 kg patient over roughly 60-120 minutes. The patient is typically monitored closely for vital signs and oxygen saturation by pulse oximetry. Blood samples may be obtained at intervals and saved for analysis. Cell re-infusions are repeated, for example, roughly every month for a total of 10-12 treatments in a one year period. After the first treatment, it may be possible to perform infusions on an outpatient basis at the discretion of the clinician.

The invention further provides a method that can test the effectiveness of an antigenic peptide. A variety of similar peptides can be administered as immunizations and the responses to each peptide compared in order to identify an amino acid sequence which will produce an optimal response. Modifications to peptides can be made based on known parameters such as, for example, HLA binding affinity, or modifications can be generated randomly and tested for immunogenic potential.

The amount of antigen to be employed in any particular composition or application will depend on the nature of the particular antigen and of the application for which it is being used, as will readily be appreciated by those of skill in the art.

The methods can be further modified by contacting the cell with an effective amount of a cytokine or co-stimulatory molecule, *in vitro* or *in vivo.* These agents can be delivered as polypeptides, proteins or alternatively, as the polynucleotides or genes encoding them. Cytokines, co-stimulatory molecules and chemokines can be provided as impure preparations (e.g., isolates of cells expressing a cytokine gene, either endogenous or exogenous to the cell) or in a "purified" form. Purified preparations are preferably at least about 90%, 95%, or at least about 99% pure.

Where both cytokine and antigen are to be delivered to an individual, they may be provided together or separately. When they are delivered as polypeptides or proteins, they can be delivered in a common encapsulation device or by means of physical association such as covalent linkage, hydrogen bonding, hydrophobic interaction, van der Waals interaction, *etc*. In an alternative embodiment, the compounds are provided together in that polynucleotides encoding both are provided. In some embodiments, both factors are expressed from a single contiguous polynucleotide, as a fusion protein in which the cytokine and the antigen are covalently linked to one another via a peptide bond. Alternatively or additionally, the genes may be linked to the same or equivalent control sequences, so that both genes become expressed within the individual in response to the same stimuli. Administration of cytokine and/or antigen may optionally be combined with the administration of any other desired immune system modulatory factor such as, for example, an adjuvant or other immunomodulatory compound.

In order to assay the ability of DCs to activate T cells, T cells may be obtained using procedures known in the art. Briefly, density gradient centrifugation can be used to separate PBMCs from red blood cells and neutrophils. T cells can be enriched by negative or positive selection with appropriate monoclonal antibodies coupled to columns or magnetic beads. Mature DCs prepared by the methods of the invention can stimulate T cells better *in vivo* than control DCs by at least 10%, 20%, 30%, 40%, 50%, 60%, 70%, 80%, 90%, or 100% or more, as measured by any suitable assay known in the art and/or described herein.

Cell surface markers can be used to identify and/or isolate particular cell types for various purposes. For example, human stem cells typically express CD34 antigen. Methods of identifying and isolating particular cell types include, for example, FACS, column chromatography, panning with magnetic beads, western blots, radiography, electrophoresis, capillary electrophoresis, high performance liquid chromatography (HPLC), thin layer chromatography (TLC), hyperdiffusion chromatography, and the like, and various immunological methods such as fluid or gel precipitin reactions, immunodiffusion (single or double), immunoelectrophoresis, radioimmunoassays (RIAs), enzyme-linked immunosorbent assays (ELISAs), immunofluorescent assays, and the like. For a review of immunological and immunoassay procedures in general, see, *e.g.*, Stites and Terr, eds.) 1991 BASIC AND CLINICAL IMMUNOLOGY (7th ed.); Harlow and Lane, eds. (1988) ANTIBODIES: A LABORATORY MANUAL; Harlow and Lane, eds. (1999) USING ANTIBODIES: A LABORATORY MANUAL.

Antibodies used for various purposes can be any suitable type, such as, for example, monoclonal or polyclonal; antibodies can be human, chimeric, or humanized. Functional fragments or derivatives of antibodies may also be used, including, for example, Fab, Fab', Fab2, Fab'2, and single chain variable regions. Antibodies can be produced in cell culture, in phage, or in any suitable animal. Antibodies can be tested for specificity of binding by comparing binding of the antibody to an appropriate antigen (*i.e.*, an antigen that is or may be recognized by the antibody) to binding of the antibody to an irrelevant antigen or antigen mixture (*i.e.*, an antigen that is not expected to be or is not recognized by the antibody) under a given set of conditions. Antibody binding is considered to be specific if the antibody binds to the appropriate antigen at least 2, 5, 7, or 10 times more than to the irrelevant antigen or antigen mixture.

A variety of techniques are known in the art for detecting and quantifying proteins and/or polypeptides and include but are not limited to radioimmunoassays, ELISA (enzyme linked immunosorbent assays), "sandwich" immunoassays, immunoradiometric and/or immunostaining assays, *in situ* immunoassays (using *e.g.*, colloidal gold, enzyme or radioisotope labels), Western blot analysis, immunoprecipitation assays, immunofluorescent assays and PAGE-SDS. Flow cytometry can be used to evaluate populations of cells for the presence or absence of various cell-surface markers. See, for example, Givan (1992) Flow Cytometry: First Principles (John Wiley & Sons, New York, NY, USA).

Methods for cryopreservation of cells are known in the art; see, *e.g.*, Feuerstein et al. (2000) J. Immunol. Meth. 245: 15-29.

The immunogenicity of the dendritic cells and the quality and quantity of the educated T cells induced and expanded by the methods of the invention can be determined by well-known methodologies including, but not limited to the following:

$^{51}$Cr-release lysis assay: Antigen-specific T-cells can be compared for their ability to lyse $^{51}$Cr-labeled targets that present antigen via peptide-pulsing or some other means, with "more active" compositions showing a greater lysis of targets as a function of time. Performance can be evaluated by the kinetics of lysis as well as the amount of lysis within a particular time period, such as, for example, 4 hours (see, *e.g.,* Ware et al. (1983) J. Immunol. 131: 1312).

Cytokine-release assay: Functional activity of T cells can also be measured by the the types and quantities of cytokines secreted by the cells upon contacting modified APCs. Cytokines can be measured by ELISA or ELISPOT assays to determine the rate and total amount of cytokine production (see, e.g., Fujihashi et al. (1993) J. Immunol. Meth. 160: 181; Tanquay and Killion (1994) Lymph. Cyt. Res. 13: 259).

*In vitro* education of T-cells: DCs can be assayed for the ability to elicit reactive T cell populations from PBMCs from normal donors or from patients. Elicited T cells can be tested for lytic activity, cytokine release, polyclonality, and cross-reactivity to the antigen (see, *e.g.*, Parkhurst et al. (1996) J. Immunol. 157: 2539-2548).

Transgenic animal models: Immunogenicity can be assessed *in vivo* by vaccinating HLA transgenic mice with the compositions of the invention and determining the nature and magnitude of the induced immune response. Alternatively, the hu-PBL-SCID mouse model allows reconstitution of a human immune system in a mouse by adoptive transfer of human PBL. These animals may be vaccinated with the compositions and analyzed for immune response as previously mentioned in Shirai et al. (1995) J. Immunol. 154: 2733; Mosier et al. (1993) Proc. Nat'l. Acad. Sci. USA 90: 2443.

Proliferation Assays: T cells will proliferate in response to reactive compositions, and proliferation can be monitored quantitatively by measuring, for example, $^3$H-thymidine uptake (see, *e.g.,* Caruso et al. (1997) Cytometry 27: 71).

Primate models: Chimpanzees share overlapping MHC-ligand specificities with human MHC molecules and so can be used to test HLA-restricted ligands for relative *in vivo* immunogenicity (see, *e.g.*, Bertoni et al. (1998) J. Immunol. 161: 4447).

Monitoring TCR Signal Transduction Events: Successful TCR engagement by MHC-ligand complexes is associated with several intracellular signal transduction events (*e.g.*, phosphorylation). These events have been correlated qualitatively and quantitatively with the relative abilities of compositions to activate effector cells through TCR engagement (see, *e.g.*, Salazar et al. (2000) Tnt. J. Cancer 85: 829; Isakov et al. (1995) J. Exp. Med. 181: 375).

In accordance with the above description, the following examples are intended to illustrate, but not limit, the various aspects of this invention.

EXPERIMENTAL

The following materials and methods were used where applicable in the examples described herein below. Collectively, the following experiments show that the introduction of a chimeric E/L selectin into DCs makes it possible for the DCs to enter lymph nodes directly from the bloodstream through high endothelial venules (HEV).

**Antibodies:** For determining the phenotypes of DCs, the following antibodies (Abs) were used: FITC-labeled anti-CD83, anti-CD86, anti-CD25, and anti-CD80 (BD Pharmingen, Heidelberg Germany); FITC-labeled anti-HLA-DR (BD Biosciences, Heidelberg, Germany); and FITC-labeled anti-HLA class I (Chemicon International, Hampshire, United Kingdom). Isotype controls were IgG1-FITC (BD Pharmingen) and IgG2a-FITC (Chemicon International). For determining E/L-selectin expression, FITC-labeled anti-human E-Selectin/CD62E mAb (Clone BBIG-E5) was used (R&D Systems GmbH, Wiesbaden-Nordenstadt, Germany). For determining the T cells' phenotype, ECD-labeled anti-CD45RA, PC7-labeled anti-CD8 (both of Beckman Coulter GmbH, Krefeld, Germany), and FITC-labeled anti-CCR7 (R&D Systems GmbH) were used.

**Human DC generation from leukapheresis and whole blood**: Monocyte-derived DCs ("moDCs") were generated essentially as described in Berger et al. (2002) J. Immunol. Meth. 268: 131-140. Briefly, peripheral blood mononuclear cells (PBMCs) were prepared from leukapheresis products or whole blood of healthy donors by density centrifugation using Lymphoprep (Axis-Shield, Oslo, Norway). Blood products were obtained following informed consent and approved by the institutional review board. PBMCs were resuspended in autologous medium that consisted of RPMI 1640 (Cambrex, Verviers, Belgium) containing 1% of heat-inactivated human plasma, 2 mM L-glutamine (Bio-Whittaker), and 20 mg/l gentamicin (Sigma-Aldrich Chemie GmbH, Taufkirchen, Germany). PBMCs were then either transferred to cell-factories (Nunc, Roskilde, Denmark) at $1.2 \times 10^9$ cells per cell-factory, or, for generation of DCs on a smaller scale, to tissue culture dishes (Falcon (BD), Le Pont De Claix, France) at $30 \times 10^6$ cells/dish. Cells were incubated for 1-2 h at 37°C to allow for adherence. The non-adherent fraction was then removed and cryopreserved, while 200 ml (cell factory) or 10 ml (dish) of autologous medium was added to the adherent cells. Feeding of cells with medium and cytokines (GM-CSF (Leukine, Berlex, Montville NJ, USA) and IL-4 (Strathmann, Hamburg, Germany)), and maturation of DC was performed essentially as described previously in Schaft et al. (2005) J. Immunol. 174: 3087-3097). After 24 hours of maturation, the cells were used for electroporation.

**Mouse BM-derived DC generation:** The generation of bone marrow (BM)-DC with GM-CSF was essentially as described in Lutz et al. (1999) J. Immunol. Meth. 223: 77-92. Cell culture medium (R10) consisted of RPMI-1640 (GIBCO BRL, Eggenstein, Germany) supplemented with penicillin (100 U/ml, Sigma, Deisenhofen, Germany), strepto-mycin (100 μg/ml, Sigma), L-glutamine (2 mM, Sigma), 2-mercaptoethanol (50 μM, Sigma), and 10% heat-inactivated and filtered FCS (FCS from PAA, Cölbe, Germany; filtered with 0.22 μm filter from Millipore, Eschborn, Germany). On day 0, BM leukocytes gained from murine hind legs were seeded at $2 \times 10^6$ cells per dish in 10 ml R10 medium containing 10% GM-CSF supernatant from a cell line transfected with the murine GM-CSF gene (Zal et al. (1994) J. Exp. Med. 180: 2089-2099). On day 3, another 10 ml R10 medium containing 10% GM-CSF supernatant were added to the plates. On day 6, half of the culture supernatant was collected and centrifuged, and the cell pellet was resuspended in 10 ml fresh R10 medium containing 10% GM-CSF supernatant and placed back into the original dish. At day 8, cells were harvested for electroporation.

**Production of *in vitro* transcribed RNA:** For *in vitro* generation of RNA two plasmids were used: the pGEM4Z64A-MelanA plasmid (Heiser et al. (2000) J. Immunol. 164: 5508-5514; kindly provided by Dr. I. Tcherepanova, which contains the full length open reading frame of the melanoma antigen Melan-A), and the PSP73 SphA64+EL plasmid (which contains the extracellular domain of human E-selectin and the transmembrane and intracellular domain of human L-selectin in one open reading frame, kindly provided by Dr. C. Robert). Both plasmids were transcribed as described before (see Schaft et al. (2005) J. Immunol. 174: 3087-3097) using an Ambion mMESSAGE mMACHINE T7 ULTRA kit (Austin, Texas, USA) according to the manufacturer's instructions

**Electroporation of dendritic cells:** Human and mouse DCs were harvested from the cell-factories or dishes and washed once with pure RPMI 1640 and once with PBS (all at room temperature). The cells were resuspended in OptiMEM without phenol-red (Gibco-BRL, Long Island, USA) at a concentration of $4\text{-}6 \times 10^7$ cells/ml (human) or $4\text{-}10 \times 10^7$ cells/ml (mouse). RNA was electroporated into DCs with a Genepulser Xcell (Biorad, Munich, Germany) machine essentially as described previously (see Schaft et al. (2005) J. Immunol. 174: 3087-3097) with modifications of time constant and RNA concentration to increase expression levels.

Particularly, it was determined that the optimal electroporation conditions for human DCs included the use of a 500V charge with a 4 mm cuvette and a 1 millisecond square wave pulse at room temperature in Optimem medium. RNA was used at a final concentration of 150 micrograms per milliliter and was incubated with the cells in the cuvette for 3 minute prior to electroporation. The optimal electroporation conditions for murine DCs included the use of a 500V charge with a 4 mm cuvette and a 2 millisecond square wave pulse at room temperature in Optimem medium. RNA was used at a final concentration of 150 micrograms per milliliter, but was not preincubated with the cells in the cuvette prior to electroporation.

Immediately after electroporation, the cells were transferred to autologous medium supplemented either with the above-indicated concentrations of GM-CSF and IL-4 (for human cells), or R10 medium supplemented with 10% GM-CSF-containing supernatant (for mouse cells; as described above in the section headed "BM-derived DC generation").

**Cryopreservation of cells:** Cryopreservation was performed essentially as described previously (see, *e.g.*, Feuerstein et al. (2000) J. Immunol. Meth. 245: 15-29). Briefly, cells were taken up in 20% human serum albumin (HSA, Pharmacia & Upjohn) at a concentration of $5\text{-}10 \times 10^6$ cells/ml (for DCs) or $20\text{-}50 \times 10^6$ cells/ml (for non-adherent cells) and stored for 10 minutes on ice. An equal volume of cryopreservation medium was added to the cell suspension (*i.e.,* 55% HSA (20%), 20% dimethyl sulfoxide (DMSO) (Sigma-Aldrich) and 25% glucose (Glucosteril 40™, Fresenius, Bad Homburg, Germany). Cells were then frozen in a cryo-freezing container (Nalgene, Roskilde, Denmark) at-1°C/min to -80°C. Thawing was performed by holding cryotubes in a 37°C water-bath until detachment of the cells was visible. Cells were then poured into 10ml of RPMI 1640 medium, washed, and added to a cell culture dish containing pre-warmed autologous medium with 250 IU IL-4/ml and 800 IU GM-CSF/ml. Cells were allowed to rest for 1-2 h in a 37°C incubator prior to further experiments.

**Flow cytometric analysis:** For surface stainings, DCs were washed and thereafter suspended at $1 \times 10^5$ cells in 100 $\mu$l of cold FACS solution (DPBS (Bio Whittaker, Walkersville, Maryland, USA) with 0.1 % sodium azide (Sigma-Aldrich) and 0.2% HSA (Octapharma, Langenfeld, Germany)) and incubated with monoclonal antibody or appropriate isotype controls for 30 min. Cells were then washed twice and resuspended in 100 $\mu$l of cold FACS solution. Stained cells were analyzed for immunofluorescence with a FACSstar cell analyzer (Becton-Dickinson). Cell debris was eliminated from the analysis using a gate on forward and side scatter. A minimum of $10^4$ cells was analyzed for each sample of surface stained cells. Results were analyzed using Cellquest software (Becton-Dickinson).

**Transwell migration assay:** Transwell migration assays were performed using transwell inserts (Costar, London, UK) with a pore size of 5 $\mu$m and CCL19 (100 ng/ml, tebu-bio GmbH, Offenbach, Germany) essentially as described previously in Schaft et al. (2005) J. Immunol. 174: 3087-3097.

**Cytotoxic T Cell (CTL) induction assay:** DCs were electroporated without RNA, with E/L-selectin RNA alone, with MelanA RNA alone, or with MelanA in combination with E/L-selectin RNA. In addition, mock-electroporated and E/L-selectin RNA-electroporated DC were pulsed for 1 h at 37˚C with 10 $\mu$g/ml of the MelanA-derived HLA-A2-binding analogue peptide ELAGIGILTV for comparison. The non-adherent cell fraction from the same healthy donor was used as the source for the generation of CD8[+] T-cells using MACS (Miltenyi Biotech, Bergisch-Gladbach, Germany) according to manufacturers' instructions. CD8[+] cells were then co-cultivated with the above described, differently pretreated, DCs at final concentrations of $1 \times 10^6$/ml and $1 \times 10^5$/ml, respectively, in RPMI supplemented with 10% pooled-serum (Cambrex), 10 mM Hepes, 1 mM sodium pyruvate, 1% MEM non-essential amino acids (100x), 2 mM L-glutamine, 20 mg/l gentamycin, and 20 U/ml of IL7. On days 2 and 4, 20 IU/ml of IL2 and 20 U/ml of IL7 were added. On day 7, the cells were harvested and analyzed.

**Tetramer staining and phenotyping of antigen-specific CD8+ T cells:** HLA-A2-MelanA tetramer-staining (Beckman Coulter GmbH) and T cell phenotype using anti-CCR7, anti-CD45RA and anti-CD8 antibodies were performed essentially as described previously in Schaft et al. (2005) J. Immunol. 174: 3087-3097. Cells were analyzed on a CYTOMICS FC500 from Beckman Coulter.

**Cytotoxicity assay:** Cytotoxicity was tested in standard 4-h $^{51}$Cr release assays essentially as described previously in Schaft et al. (2005) J. Immunol. 174: 3087-3097.

**E/L-selectin-induced *in vitro* migration assay:** Dendritic cells were electroporated with or without E/L-selectin RNA and resuspended at a concentration of $1 \times 10^6$ cells/ml. Rectangular cover slips (24 x 60 mm) were coated with 5 $\mu$g biotinylated polyacrylamide bound to sialyl-Lewis[x] and sulphated tyrosine (Lectinity Holdings Inc., Moscow, Russia) and air-dried. All cover slips were incubated for at least 30 minutes with 0.5% bovine serum albumin (in PBS) to prevent non-specific binding. Transparent flow chambers with a slit depth of 50 $\mu$m and a slit width of 500 $\mu$m and equipped with coated or uncoated cover slips were briefly rinsed with Hank's balanced salt solution (HBSS) supplemented with 2 mM CaCl$_2$ before connection to a syringe containing 1 ml cell suspension. Perfusion was performed at 20˚C using a pulse-free pump at a shear rate of 1.04 dyne/s$^2$. During perfusion, microscopic phase-contrast images were recorded in real time. After 10 minutes of perfusion, four different microscopic fields (10x objective) were recorded and the numbers of adhering cells were counted for each field. Image analysis was performed off-line using MetaView Imaging software (Universal Imaging Corporation, Downington, USA).

**E/L-selectin-induced *in vivo* migration:** Murine DCs were electroporated with or without E/L-selectin RNA and stained with 5-chloromethylfluorescein diacetate (CMFDA) (Molecular Probes, OR, USA) according to the manufacturer's guidelines. Cells were injected into the tail vein ofC57/B6 mice; sixteen hours later, the mice were sacrificed and the inguinal lymph node (LN), the spleen and parts of the lung were extracted and immediately frozen in liquid nitrogen. The organs were embedded in Tissue-Tek O.C.T. Compound (Sakura, NL) and stored at -80˚C. The frozen organs were then cut into 10 $\mu$m thick sections with a Leica CM3050 S Cryostat (Leica, Wetzlar, Germany) and stored on SuperFrost Plus microscope slides (Menzel GmbH, Braunschweig, Germany) at -20˚C.

For immunofluorescence stainings, the frozen sections were thawed and dried for 10 minutes. Sections were fixed with 4% paraformaldehyde (Merck, Darmstadt, Germany) for 20 minutes. After washing with PBS and neutralizing of excessive aldehyde groups with 0.1 M glycine for 15 minutes, the sections were incubated for 15 minutes with a blocking solution of 2% BSA (PAA, Cölbe, Germany) and stained with an antibody specific for CD90.2 (Thy1.2, BD Bioscience, Heidelberg, Germany) diluted at 1:200 in 2% BSA solution. After 30 minutes, the cells were incubated with an anti-rat Alexa555-conjugated antibody (Invitrogen GmbH, Karlsruhe, Germany) diluted at 1:1000 in 2% BSA. After 30 minutes of incubation, the sections were embedded in Fluoromount mounting medium. (Serva Electrophoresis GmbH, Heidelberg, Germany). Fluorescence analysis was performed with a Leica DMRD research microscope. (Leica, Wetzlar, Germany).

**Example 1: Electroporation of E/L-selectin-encoding RNA into mature human DCs results in high transfection**

**efficiency and yields**

RNA encoding chimeric E/L-selectin was electroporated into DC using the optimized electroporation protocol established after testing various electroporation settings described above. RNA-transfected DCs were cryopreserved 4h after electroporation, thawed, and evaluated. As shown in Figure 1 (Panel a), these DCs exhibited a high and homogenous expression of E/L-selectin. High expression was observed even after 24h and 48 h after the DCs were thawed (Figure 1a), proving that a prolonged expression of E/L-selectin was obtained.

In order to use DCs as vaccines in human patients, it is critical that DCs survive the preparation process. DCs must survive the electroporation and cryopreservation steps and should not exhibit undue damage (*e.g.*, toxic effects) from expressing the homing peptide and any antigens of interest. Thus, DCs were evaluated for yield (*i.e.,* the percentage of living cells after electroporation and cryopreservation, in comparison to the number of cells prior to the whole procedure). DCs electroporated with E/L-selectin RNA or without RNA were cryopreserved and then thawed. Yield was determined by counting in trypan-blue. Cells were evaluated at 0 hours after thawing or were cultured in autologous medium with IL-4 and GM-CSF at 37° C and evaluated at 24 hours or 48 hours after thawing. As shown in Figure 1 (Panel b), survival of DCs immediately after thawing was about 80% and decreased slowly over 48 hours. Similar results were obtained with control DCs, which indicated that the introduction of E/L-selectin RNA had no toxic effect on the DCs.

**Example 2: Mature Human DCs Electroporated with E/L-selectin RNA Show Normal Marker Phenotype and CCR7-Mediated Migration**

For optimal performance, a DC vaccine should comprise the highest possible frequency of mature DCs that are capable of presenting tumor-associated antigens ("TAA"). Thus, the electroporated DCs were evaluated to determine whether they had retained their mature phenotype in whole or in part. DCs electroporated with ELS RNA or that had been mock-electroporated were evaluated for surface expression of CD80, CD83, CD86, CD25, HLA class I and HLA-DR molecules. A mature phenotype was detected and no difference in both DC populations was observed; results are shown in Figure 2.

For optimal performance, DCs should also retain CCR7-mediated migration capacity, which is critical for normal DC migration from the skin into the lymph nodes. Furthermore, functional CCR7 expression is also necessary for DCs to "roll" on HEV. Accordingly, DCs that were electroporated with ELS RNA or were mock-electroporated were compared. Migration capacity was tested in a standard transwell *in vitro* migration assay (see, *e.g.,* Scandella et al. (2002) Blood 100: 1354-1361). In this assay, DCs were placed in the upper well of the transwell system; the chemokine CCL19 was then placed either in the upper well (*i.e.,* in the same well as the cells) or in the lower well. Cells which were placed in the upper well with the chemokine but migrated away from that well are said to have migrated "against" the chemokine, while cells placed in a different well from the chemokine but which migrate toward the chemokine well are said to have migrated "towards" the chemokine. Cells were allowed to migrate for 2 hours and then evaluated. Neither group of DCs migrated against the CCL19 gradient, and most importantly, both populations of DCs showed an identical migration capacity towards CCL19 (Figure 3). As a negative control, cells were incubated in transwells without CCL19.

As illustrated by the above experiments, several properties of DCs are unaffected by expression of E/L-selectin on the surface of the DC, including the mature marker phenotype and the CCR7- mediated migratory capacity.

**Example 3: DCs electroporated with E/L-selectin RNA are still efficient inducers of MelanA-specific CTL**

For optimal performance of a DC vaccine against cancer, DCs should be able to efficiently induce TAA-specific CTL. To test this, we chose the melanoma-associated antigen (Ag) MelanA. The MelanA Ag works well for this assay because MelanA-specific T cells are relatively frequent and of a naive phenotype in volunteers. These T cells can be detectably expanded even after one stimulation if potent DCs are used (see, *e.g.,* Schaft et al. (2005) J. Immunol. 174: 3087-3097; Pittet et al. (1999) J. Exp. Med. 190: 705-715; Romero et al. (2002) Immunol. Rev. 188: 81-96).

The induction capacity of various DCs was then assayed. The induction capacity of DCs loaded with MelanA alone was compared to the induction capacity of DCs that had been loaded with MelanA and electroporated with E/L-selectin RNA. Antigen loading was accomplished either by co-electroporation of RNA encoding native MelanA or by pulsing with a MelanA derived HLA-A2 restricted analogue peptide, which provides better stimulation than the natural MelanA peptide under these circumstances (see, *e.g.,* Schaft et al. (2005) J. Immunol. 174: 3087-3097; Abdel-Wahab et al. (2003) Cell. Immunol. 224: 86-97).

Electroporated DCs were co-cultivated for one week with autologous CD8[+] T cells, and the percentage of HLA-A2/MelanA-tetramer positive T cells was determined. Tetramer-positive T cells were also classified into one of four phenotypes by their CCR7 and CD45RA expression (see, *e.g.*, Sallusto et al. (1999) Nature 401: 708-712): naive, central memory, effector memory, and lytic effectors. DCs electroporated with MelanA RNA alone as well as with the combination of MelanA RNA and E/L-selectin RNA were able to expand the pool of MelanA-specific T cells to the same extent, with a strong bias towards the effector population (Figure 4a). Mock-electroporated and E/L-selectin RNA-electroporated DCs served as negative controls. The DCs loaded with MelanA analogue peptide stimulated antigen-specific T cells more strongly, but again no substantial difference in stimulation capacity was observed between the DCs that had been electroporated with ELS RNA and the DCs that had been mock-electroporated (Figure 4a).

The T cells generated by this procedure were checked for their cytolytic capacity in a standard $Cr^{51}$-release assay using T2 cells loaded with the specific analogue peptide as target cells. T cells that had been stimulated with MelanA analogue peptide-loaded DCs showed high cytolysis (Figure 4, panel b) whether those DCs had also been electroporated with ELS RNA or mock-electroporated. T cells also showed similar lytic capacities whether they were stimulated by DCs that had been electroporated with MelanA RNA alone or by DCs that had been electroporated with a combination of ELS RNA and MelanA RNA (Figure 4, panel b). T cells did not show cytolysis when they had been stimulated with DCs that had been electroporated with ELS RNA or mock-electroporated (Figure 4, panel b). Background lysis of T2 target cells loaded with an irrelevant peptide was <10% for all T cell populations tested (data not shown).

In summary, these data show that coexpression of E/L-selectin by DCs did not inhibit the DCs' functional ability to induce CTLs by presenting a tumor-associated Ag, and that coexpression of MelanA by DCs did not inhibit membrane expression of E/L selectin.

**Example 4: DCs electroporated with E/L-selectin RNA roll by binding to sialyl-Lewis^x**

The functionality of the introduced chimeric E/L-selectin protein was confirmed in an *in vitro* rolling assay using slides coated with sialyl-Lewis^x (SLX) in a parallel plate flow chamber. The shear force in the assay approximated the shear force in high endothelial venules ("HEV"), *e.g.,* 1.04 dyne/s²). DCs electroporated with E/L-selectin RNA rolled on the SLX-coated slides with a slow rolling velocity, while mock-electroporated DCs did not roll or stick on SLX-coated slides. Neither population of DCs rolled on or stuck to uncoated slides. After 10 minutes of flow, pictures of four random fields were taken, and cells were counted; this data is summarized for three different DC donors in Figure 5 and p-values are given, indicating statistical significance of the data (Student's T-test). For DCs from all three donors, DCs electroporated without RNA did not stick to or roll on the SLX-coated slides at all (Figures 5 and 7), while DCs electroporated with E/L-selectin RNA were observed to both stick and roll (Figure 5). Introduction of E/L-selectin into mouse DC did also result in rolling of these DC on SLX-coated slides.

Taken together, these data show that DCs transfected with E/L-selectin RNA functionally expressed the chimeric E/L-selectin protein as demonstrated by *in vitro* rolling.

**Example 5: DCs electroporated with E/L-selectin RNA efficiently extravasate from the blood and migrate to lymph nodes *in vivo***

To demonstrate the *in vivo* functionality of the introduced E/L-selectin, mouse DCs (C57/B6) were electroporated with E/L-selectin RNA, stained with CMFDA, and injected into the tail vein of C57/B6 mice. After 14h-18h, the mice were sacrificed, and cryosections of spleen and lymph nodes were made. As shown in Figure 6, positively-staining DCs were detected in the spleen following injection of DCs whether those DCs had been electroporated with ELS RNA or whether they had been mock-electroporated. However, only DCs that had been electroporated with ELS RNA migrated into the peripheral lymph nodes, while mock-electroporated DC did not (Figure 6), proving that DCs expressing E/L-selectin had gained the ability to transmigrate from the blood into the lymph node.

In aggregate, these data show that RNA electroporation can be used to provide functional expression of E/L selectin by DCs, *i.e.,* to provide DCs that can migrate from the blood to the lymph node following intravenous administration.

Throughout this disclosure, various publications, patents and published patent specifications are referenced by an identifying citation. Each referenced publication, patent, and published patent specification is hereby specifically incorporated by reference into the present disclosure to more fully describe the state of the art to which this invention pertains.

**Claims**

1. A composition comprising a dendritic cell which has been transiently transfected with RNA encoding a membrane homing polypeptide, wherein said dendritic cell can roll on a surface coated with a ligand for said polypeptide.

2. The composition of claim 1, wherein said membrane homing polypeptide is a selectin, preferably said selectin is E-selectin, L-selectin, P-selectin, or a chimera thereof, most preferably said selectin is an E/L selectin chimera.

3. The composition of claim 1, wherein

(i) said dendritic cell rolls on a surface coated with sialyl-Lewis$^x$; and/or
(ii) said dendritic cell is mature; and/or
(iii) said dendritic cell is a monocyte-derived dendritic cell; and/or
(iv) said dendritic cell is a human dendritic cell; and/or
(v) said dendritic cell has been transiently transfected by RNA electroporation.

4. The composition of claim 1, wherein said dendritic cell is additionally loaded with an antigen of interest.

5. The composition of claim 4, wherein said antigen is loaded into the dendritic cell by a method selected from the group consisting of pulsing and transfection.

6. The composition of claim 4 or 5, wherein said antigen

(i) is a tumor associated antigen; or
(ii) is a pathogen-specific antigen, preferably said pathogen is HIV or HCV.

7. A vaccine comprising the composition according to any one of claims 1 to 6.

8. Use of the composition according to any one of claims 1 to 6 for the manufacture of a medicament for the treatment of cancer.

9. The use of claim 8, wherein the medicament is suitable for intravenous administration.

10. A method of transfecting dendritic cells with RNA comprising electroporating mature dendritic cells in the presence of RNA at a field strength between 100 Volts/mm and 150 Volts/mm and a square wave pulse length of between 0.8 ms and 2 ms.

11. A method of delivering dendritic cells to a lymph node in a human subject comprising the steps of:

a) providing isolated dendritic cells which have been transiently transfected with RNA, wherein said RNA encodes a membrane homing polypeptide; and
b) intravenously administering the dendritic cells to the human subject.

12. The method of claim 11, wherein said dendritic cells are antigen-loaded.

13. The method of claim 11, wherein said dendritic cells were originally isolated from the human subject.

14. The method of claim 11, wherein said membrane homing polypeptide is a selectin.

15. The method of claim 14, wherein said selectin is an E/L selectin chimera.

16. The method of claim 11, wherein said isolated dendritic cells have further been transiently transfected with RNA encoding a tumor-associated antigen.

17. The method of claim 11, wherein said isolated dendritic cells have further been transiently transfected with RNA encoding a pathogen-specific antigen.

18. The method of claim 17, wherein said pathogen-specific antigen is an antigen from HIV or HCV.

Fig. 1

**Fig. 2**

marker expression

# Fig. 3

Fig. 4

Fig. 5

Fig. 6

spleen      lymph node

No RNA

ELS RNA

**European Patent Office**

## PARTIAL EUROPEAN SEARCH REPORT

which under Rule 45 of the European Patent Convention shall be considered, for the purposes of subsequent proceedings, as the European search report

**Application Number**

EP 06 11 0306

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SAEBOE-LARSSEN S ET AL: "mRNA-based electrotransfection of human dendritic cells and induction of cytotoxic T lymphocyte responses against the telomerase catalytic subunit (hTERT)" JOURNAL OF IMMUNOLOGICAL METHODS, ELSEVIER SCIENCE PUBLISHERS B.V.,AMSTERDAM, NL, vol. 259, no. 1-2, 1 January 2002 (2002-01-01), pages 191-203, XP004324213 ISSN: 0022-1759 * figure 2 *  ----- | 10 | INV. A61K35/12 A61K48/00 C12N15/63 C12N15/87 A61P35/00 |
| D,Y | ROBERT C ET AL: "Gene therapy to target dendritic cells from blood to lymph nodes." GENE THERAPY, vol. 10, no. 17, August 2003 (2003-08), pages 1479-1486, XP002392007 ISSN: 0969-7128 * the whole document *  -----  -/-- | 1-9, 11-18 | |
| | | | TECHNICAL FIELDS SEARCHED (IPC)  A61K C12N A61P |

### INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| The Hague | 27 July 2006 | Kools, P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

**European Patent Office**

## PARTIAL EUROPEAN SEARCH REPORT

Application Number

EP 06 11 0306

| | DOCUMENTS CONSIDERED TO BE RELEVANT | | CLASSIFICATION OF THE APPLICATION (IPC) |
|---|---|---|---|
| Category | Citation of document with indication, where appropriate, of relevant passages | Relevant to claim | |
| D,Y | WO 00/60055 A (THE BRIGHAM AND WOMEN'S HOSPITAL, INC; THE CENTER FOR BLOOD RESEARCH,) 12 October 2000 (2000-10-12) * the whole document * ----- | 1-9, 11-18 | |
| Y | LUNDQVIST A ET AL: "Nonviral and viral gene transfer into different subsets of human dendritic cells yield comparable efficiency of transfection" JOURNAL OF IMMUNOTHERAPY, LIPPINCOTT WILLIAMS & WILKINS, HAGERSTOWN, MD, US, vol. 25, no. 6, 2002, pages 445-454, XP002983187 ISSN: 1524-9557 * abstract * ----- | 1-9, 11-18 | |
| A | KIEFFER J D ET AL: "Neutrophils, monocytes, and dendritic cells express the same specialized form of PSGL-1 as do skin-homing memory T cells: Cutaneous lymphocyte antigen" BIOCHEMICAL AND BIOPHYSICAL RESEARCH COMMUNICATIONS, ACADEMIC PRESS, SAN DIEGO, CA, US, vol. 285, no. 3, 20 July 2001 (2001-07-20), pages 577-587, XP002291379 ISSN: 0006-291X * the whole document * ----- | 1-18 | TECHNICAL FIELDS SEARCHED (IPC) |
| A | CAVANAGH LOIS L ET AL: "Activation of bone marrow-resident memory T cells by circulating, antigen-bearing dendritic cells" NATURE IMMUNOLOGY, vol. 6, no. 10, October 2005 (2005-10), pages 1029-1037, XP002392008 ISSN: 1529-2908 * the whole document * ----- | 1-18 | |

EPO FORM 1503 03.82 (P04C10)

**European Patent Office**

**INCOMPLETE SEARCH SHEET C**

Application Number

EP 06 11 0306

Although claims 11-18 are directed to a method of treatment of the human/animal body (Article 52(4) EPC), the search has been carried out and based on the alleged effects of the compound/composition.

-----

**ANNEX TO THE EUROPEAN SEARCH REPORT
ON EUROPEAN PATENT APPLICATION NO.**

EP 06 11 0306

This annex lists the patent family members relating to the patent documents cited in the above-mentioned European search report.
The members are as contained in the European Patent Office EDP file on
The European Patent Office is in no way liable for these particulars which are merely given for the purpose of information.

27-07-2006

| Patent document cited in search report | Publication date | Patent family member(s) | Publication date |
|---|---|---|---|
| WO 0060055 A | 12-10-2000 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6929792 B
- WO 9729182 A
- US 5199942 A
- WO 9528479 A
- EP 0922758 A
- EP 0633930 B
- US 20040152191 A
- RU 41740
- US 20040146492 A
- US 20040038398 A
- US 05036304 W
- US 6475483 B
- US 5538724 A
- US 6395882 B
- US 5484891 A
- US 4683195 A
- US 4800159 A
- US 4754065 A
- US 4683202 A
- US 20040072347 A
- US 20030235908 A
- US 05053271 W


**Non-patent literature cited in the description**

- Cancer Facts and Figures. *American Cancer Society,* 2004
- **FIGDOR et al.** *Nat. Med.,* 2004, vol. 10, 475
- **MARKIEWICZ et al.** *Cancer Invest.,* 2004, vol. 22, 417-434
- **GILBOA ; VIEWEG.** *Immunol. Rev.,* 2004, vol. 199, 251-263
- **PACZESNY et al.** *Semin. Cancer Biol.,* 2003, vol. 13, 439-447
- **BANCHEREAU et al.** *Cell,* 2001, vol. 106, 271-274
- **FIGDOR et al.** *Nat. Med.,* 2004, vol. 10, 475-480
- **RIDOLFI et al.** *J. Transl. Med.,* 2004, vol. 2, 27
- **DE VRIES et al.** *Cancer Res.,* 2003, vol. 63, 12-17
- **MORSE et al.** *Cancer Res.,* 1999, vol. 59, 56-58
- **ROSENBERG et al.** *Nat. Med.,* 2004, vol. 10, 909-915
- **VON ANDRIAN et al.** *New Engl. J. Med.,* 2000, vol. 343, 1020-1034
- **ROBERT et al.** *New Engl. J. Med.,* 1999, vol. 341, 1817-1828
- **CAMPBELL et al.** *J. Exp. Med.,* 2002, vol. 195, 135-141
- **DUDDA et al.** *J. Immunol.,* 2004, vol. 172, 857-863
- **DUDDA et al.** *Eur. J. Immunol.,* 2005, vol. 35, 1056-1065
- **NESTLE et al.** *Nat. Med.,* 1998, vol. 4, 328-332
- **THURNER et al.** *J. Exp. Med.,* 1999, vol. 190, 1669-1678
- **BANCHEREAU et al.** *Cancer Res.,* 2001, vol. 61, 6451-6458
- **FONG et al.** *J. Immunol.,* 2001, vol. 166, 4254-4259
- **YONEYAMA et al.** *Int. J. Hematol.,* 2005, vol. 3, 204-207
- Immunobiology. Garland Publishing, 2001
- **ROBERT et al.** *Gene Ther.,* 2003, vol. 10, 1479-1486
- **HAVIERNIK ; BUNTING.** *Curr. Gene Ther.,* 2004, vol. 4, 263-276
- **BUCKLEY.** *Lancet,* 2002, vol. 360, 1185-1186
- **HACEIN-BEY-ABINA et al.** *New Engl. J. Med.,* 2002, vol. 346, 1185-1193
- **MARSHALL.** *Science,* 2002, vol. 298, 34-35
- **ARDESHNA et al.** *Br. J. Haematol.,* 2000, vol. 108, 817-824
- **DULLAERS et al.** *Mol. Ther.,* 2004, vol. 10, 768-779
- **BRECKPOT et al.** *J. Gene Med.,* 2003, vol. 5, 654-667
- **SUMIMOTO et al.** *J. Immunol. Meth.,* 2002, vol. 271, 153-165
- **KOROKHOV et al.** *Cancer Biol. Ther,* 2005, vol. 4, 289-294
- **OPHORST et al.** *Vaccine,* 2004, vol. 22, 3035-3044
- **REA et al.** *J. Immunol.,* 2001, vol. 166, 5236-5244
- **CHO et al.** *Vaccine,* 2003, vol. 22, 224-236

- **LUNDQVIST et al.** *J. Immunother.,* 2005, vol. 28, 229-235
- **VAN TENDELOO et al.** *Gene Ther.,* 1998, vol. 5, 700-707
- **SCHAFT et al.** *J. Immunol.,* 2005, vol. 174, 3087-3097
- **ABDEL-WAHAB et al.** *J. Surg. Res.,* 2005, vol. 124, 264-273
- **DANNULL et al.** *Blood,* 2005, vol. 105, 3206-3213
- **GRUNEBACH et al.** *Cancer Gene Ther.,* 2005, vol. 12, 749-756
- **CISCO et al.** *J. Immunol.,* 2004, vol. 172, 7162-7168
- **ROBERT et al.** *Gene Therapy,* 2003, vol. 10, 1479-1486
- **STEINMAN.** *Ann. Rev. Immunol.,* 1991, vol. 9, 271-296
- **JONULEIT et al.** *Exp. Med.,* 2000, vol. 192, 1213
- **DHODAPKAR et al.** *Exp. Med.,* 2001, vol. 193, 233
- **ROMANI et al.** *J. Exp. Med.,* 1994, vol. 180, 83
- **CAUX et al.** *J. Exp. Med.,* 1996, vol. 184, 695-706
- **SALLUSTO ; LANZAVECCHIA.** *J. Exp. Med.,* 1994, vol. 179, 1109
- **BERGER et al.** *J. Immunol. Meth.,* 2002, vol. 268, 131-140
- **ROMANI et al.** *J. Immunol. Meth.,* 1996, vol. 196, 137-151
- **BENDER et al.** *J. Immunol. Meth.,* 1996, vol. 196, 121
- **JONULEIT et al.** *Eur. J. Immunol.,* 1997, vol. 27, 3135
- **INABA et al.** *J. Exp. Med.,* 1992, vol. 176, 1693-1702
- **PACZESNY et al.** *J. Exp. Med.,* 2004, vol. 199, 1503-11
- **HO et al.** *Stem Cells,* 1995, vol. 13, 100-105
- **BRENNER.** *J. Hematother.,* 1993, vol. 2, 7-17
- **YU et al.** *Proc. Nat'l. Acad. Sci.,* 1995, vol. 92, 699-703
- **SZABOLCS et al.** *J. Immunol.,* 1995, vol. 154, 5851-5861
- **SAMBROOK et al.** MOLECULAR CLONING: A LABORATORY MANUAL. 1989
- CURRENT PROTOCOLS IN MOLECULAR BIOLOGY. 1987
- METHODS IN ENZYMOLOGY. Academic Press, Inc,
- PCR: A PRACTICAL APPROACH. IRL Press at Oxford University Press, 1991
- PCR 2: A PRACTICAL APPROACH. 1995
- ANTIBODIES: A LABORATORY MANUAL. 1988
- USING ANTIBODIES: A LABORATORY MANUAL. 1999
- ANIMAL CELL CULTURE. 1987
- **ROSEN et al.** *Am. J. Pathol.,* 2005, vol. 166, 935-944
- **STREETER et al.** *J. Cell. Biol.,* 1988, vol. 107, 1853
- **SMALLEY ; LEY.** *J. Cell. Mol. Med.,* 2005, vol. 9, 255-266
- **SCHWARTZ.** *Science,* 1990, vol. 248, 1349-1356
- **JENKINS.** *Immunol. Today,* 1992, vol. 13, 69-73
- **LIU et al.** *J. Exp. Med.,* 1992, vol. 175, 437-445
- **NAUJOKAS et al.** *Cell,* 1993, vol. 74, 257-268
- **VAN SEVENTER.** *J. Immunol.,* 1990, vol. 144, 4579-4586
- **SCHWARTZ.** *Cell,* 1992, vol. 71, 1065-1068
- **FREEMAN et al.** *Science,* 1993, vol. 262, 909-911
- **YOUNG et al.** *J. Clin. Invest.,* 1992, vol. 90, 229
- **NABAVI et al.** *Nature,* 1992, vol. 360, 266-268
- **QIN ; GUNNING.** *J. Biochem. Biophys. Meth.,* 1997, vol. 36, 63-72
- **YANG.** *Arch. Virol,* 2004, vol. 149, 303-321
- **VIVINUS et al.** *Eur. J. Biochem.,* 2001, vol. 268, 1908-1917
- **STEIN et al.** *Mol. Cell. Biol.,* 1998, vol. 18, 3112-3119
- **ROBERTS ; BORIS-LAWRIE.** *J. Virol.,* 2000, vol. 74, 8111-8118
- **GALLIE et al.** *Gene,* 1995, vol. 165, 233-238
- **NIEPEL ; GALLIE.** *J. Virol.,* 1999, vol. 73, 9080-9088
- **GALLIE.** *J. Virol.,* 2001, vol. 75, 12141-12152
- **SCHLESINGER ; DUBENSKY.** *Curr. Opin. Biotechnol.,* 1999, vol. 5, 434-439
- **ZAKS et al.** *Nat. Med.,* 1999, vol. 7, 823-827
- **KARLIN ; ALTSCHUL.** *Proc. Natl. Acad. Sci. USA,* 1990, vol. 87, 2264
- **KARLIN ; ALTSCHUL.** *Proc. Natl. Acad. Sci. USA,* 1993, vol. 90, 5873-5877
- **NEEDLEMAN ; WUNSCH.** *J. Mol. Biol.,* 1970, vol. 48, 443-453
- PCR: THE POLYMERASE CHAIN REACTION. Birkhauser Press, 1994
- Martin REMINGTON'S PHARM. SCL. Mack Publ. Co, 1990
- **SHAW et al.** *Infect. Immun.,* 2002, vol. 70, 1097-1105
- **STRATOV et al.** *Curr. Drug Targ.,* 2004, vol. 5, 71-88
- **SIMON et al.** *Infect. Immun.,* 2003, vol. 71, 6372-6380

- **ENCKE et al.** *J. Immunol.,* 1998, vol. 161, 4917-4923
- **STEWART et al.** Solid Phase Peptide Synthesis. Pierce Chemical Co, 1984
- **SANTIN et al.** *Curr. Pharm. Des.,* 2005, vol. 11, 3485-3500
- **RIMOLDI et al.** *J. Immunol.,* 2000, vol. 165, 7253-7261
- **WATARI et al.** *FEBS Lett.,* 2000, vol. 466, 367-371
- **ENGELHARD et al.** *Cancer J.,* 2000, vol. 6, S272-S280
- **CHAKRABORTY et al.** *Cancer Immunol. Immunother,* 2003, vol. 52, 497-502
- **ROMERO et al.** *Immunol. Rev.,* 2002, vol. 188, 81-96
- **KORBLING et al.** *Blood,* 1986, vol. 67, 529-532
- **HAAS et al.** *Exp. Hematol.,* 1990, vol. 18, 94-98
- BASIC AND CLINICAL IMMUNOLOGY. 1991
- **GIVAN.** Flow Cytometry: First Principles. John Wiley & Sons, 1992
- **FEUERSTEIN et al.** *J. Immunol. Meth.,* 2000, vol. 245, 15-29
- **WARE et al.** *J. Immunol.,* 1983, vol. 131, 1312
- **FUJIHASHI et al.** *J. Immunol. Meth.,* 1993, vol. 160, 181
- **TANQUAY ; KILLION.** *Lymph. Cyt. Res.,* 1994, vol. 13, 259
- **PARKHURST et al.** *J. Immunol.,* 1996, vol. 157, 2539-2548
- **SHIRAI et al.** *J. Immunol.,* 1995, vol. 154, 2733
- **MOSIER et al.** *Proc. Nat'l. Acad. Sci. USA,* 1993, vol. 90, 2443
- **CARUSO et al.** *Cytometry,* 1997, vol. 27, 71
- **BERTONI et al.** *J. Immunol.,* 1998, vol. 161, 4447
- **SALAZAR et al.** *Tnt. J. Cancer,* 2000, vol. 85, 829
- **ISAKOV et al.** *J. Exp. Med.,* 1995, vol. 181, 375
- **LUTZ et al.** *J. Immunol. Meth.,* 1999, vol. 223, 77-92
- **ZAL et al.** *J. Exp. Med.,* 1994, vol. 180, 2089-2099
- **HEISER et al.** *J. Immunol.,* 2000, vol. 164, 5508-5514
- **SCANDELLA et al.** *Blood,* 2002, vol. 100, 1354-1361
- **PITTET et al.** *J. Exp. Med.,* 1999, vol. 190, 705-715
- **ABDEL-WAHAB et al.** *Cell. Immunol.,* 2003, vol. 224, 86-97
- **SALLUSTO et al.** *Nature,* 1999, vol. 401, 708-712